(19) Europäisches Patentamt
European Patent Office
Office européen des brevets

(11) **EP 3 166 575 B1**

(12) **FASCICULE DE BREVET EUROPEEN**

(45) Date de publication et mention de la délivrance du brevet:
**15.04.2020 Bulletin 2020/16**

(21) Numéro de dépôt: **15733447.5**

(22) Date de dépôt: **01.07.2015**

(51) Int Cl.:
*A61K 8/25* *(2006.01)*      *A61K 8/34* *(2006.01)*
*A61K 8/60* *(2006.01)*      *A61Q 13/00* *(2006.01)*
*A61Q 15/00* *(2006.01)*      *A61K 8/73* *(2006.01)*
*A61K 8/891* *(2006.01)*      *A61K 8/895* *(2006.01)*
*A61K 8/11* *(2006.01)*

(86) Numéro de dépôt international:
**PCT/EP2015/065005**

(87) Numéro de publication internationale:
**WO 2016/005246 (14.01.2016 Gazette 2016/02)**

(54) **COMPOSITION SOFT SOLID ANHYDRE A BASE DE PARTICULES ENCAPSULANT UN AGENT BENEFIQUE**

WASSERFREIE WEICHE FESTSTOFFZUBEREITUNG BEZOGEN AUF PARTIKELN ENTHALTEND VERKAPSELTER PFLEGESTOFF

ANHYDROUS SOFT SOLID COMPOSITION BASED ON PARTICLES ENCAPSULATING A BENEFIT AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.07.2014 FR 1456631**

(43) Date de publication de la demande:
**17.05.2017 Bulletin 2017/20**

(73) Titulaire: **L'Oréal**
**75008 Paris (FR)**

(72) Inventeurs:
• MALLE, Gérard
  F-77580 Villiers S/morin (FR)
• LUUKAS, Tiina
  F-93270 Sevran (FR)
• LAVERRE, Didier
  F-94152 Chevilly La Rue (FR)
• BARA, Isabelle
  F-94210 La Varenne St Hilaire (FR)

(74) Mandataire: **L'Oreal**
**Service D.I.P.I.**
**9, rue Pierre Dreyfus**
**92110 Clichy (FR)**

(56) Documents cités:
DE-A1-102008 035 013      JP-A- 2008 156 236
US-A- 5 508 259      US-A1- 2004 029 750
US-B1- 6 200 949

• MARTIN ET AL: "Encapsulation and Co-Precipitation Processes with Supercritical Fluids:Applications with Essential Oils", THE OPEN CHEMICAL ENGINEERING JOURNAL,, vol. 4, 1 janvier 2010 (2010-01-01), pages 31-41, XP002737531,

EP 3 166 575 B1

**Description**

La présente invention concerne une composition anhydre comprenant:

1) au moins des particules comprenant un cœur contenant au moins un agent bénéfique et une enveloppe entourant le cœur ; ladite enveloppe comprenant au moins un polysaccharide modifié hydrophobe choisi parmi les $C_5$-$C_{20}$-alcényl succinates d'amidon et au moins un glucide hydrosoluble choisi parmi les maltodextrines ayant un Dextrose Equivalent allant de 4 à 20 ; lesdites particules présentant simultanément une densité de poudre versée allant de 300,0g/l à 600,0g/l et une densité absolue supérieure à 1,0 ; lesdites particules étant sphériques et ayant un diamètre moyen en nombre allant de 1 à 30 $\mu$m et un diamètre moyen en volume allant de 5 à 150 $\mu$m et

2) au moins une phase grasse comprenant au moins un corps gras solide et au moins une huile ;

ladite composition ayant une dureté mesurée à 32°C sous une humidité de 40% allant de 15kPa à 150kPa et de préférence allant de 20kPa à 100kPa.

L'invention concerne également un procédé cosmétique de soin et/ou d'hygiène et/ou de de conditionnement et/ou de parfumage et/ou de maquillage d'une matière kératinique consistant à appliquer sur ladite matière kératinique une composition telle que définie précédemment.

L'invention concerne également un procédé de traitement cosmétique des odeurs corporelles et éventuellement de la transpiration humaine consistant à appliquer sur une matière kératinique une composition telle que définie précédemment comprenant au moins un actif déodorant et/ou un actif anti-transpirant sous forme libre (non encapsulé) et/ou sous forme encapsulée.

De nombreuses formes galéniques permettent de délivrer des agents bénéfiques notamment des produits cosmétiques ou pharmaceutiques, des produits dans la parfumerie, des produits vétérinaires notamment des produits d'hygiène et/ou le soin des animaux ; des produits d'entretien ménager comme les produits pour le soin du linge (détachants), les produits d'entretien des appareils électroménagers, des produits d'entretien des sols, carrelages, bois etc ; des produits d'entretien des matières textiles, des produits d'entretien pour la maroquinerie comme les chaussures, les semelles; des produits d'entretien dans l'industrie automobile.

Parmi celles-ci, les compositions « soft solid » (solide/mou) constituent une catégorie de produits appréciée par les consommateurs pour leur efficacité et notamment dans l'industrie cosmétique pour leurs qualités sensorielles (toucher sec et doux). Elles s'apparentent à des compositions solides qui se ramollissent sous l'effet d'une contrainte comme l'étalement sur la surface de la peau ou par exemple par extrusion à travers un dispositif à paroi ajourée (grille).

Elles sont notamment utilisées dans le domaine des cosmétiques en particulier dans le domaine des déodorants et anti-transpirants mais peuvent également être valorisées dans d'autres domaines cosmétiques tels que des produits pour le soin des matières kératiniques comme la peau, le cheveu ou les lèvres comme des produits de massage, des baumes, des pommades, des crèmes ou des gels destinés à une application topique sur la peau ou sur les cheveux..

Le but de la présente invention est de proposer de nouvelles compositions de type « soft solid » anhydres comprenant au moins un agent bénéfique encapsulé dans des particules qui seraient étanches à l'abri de l'humidité c'est-à-dire inodores si l'actif est un parfum

- les dites particules présentant une densité de poudre versée faible pour faciliter leur formulation
- lesdites particules devant également être compatibles avec les ingrédients habituels de ces formulations et suffisamment résistantes pour pouvoir être formulées en « soft solid » sans être endommagées
- ledit agent bénéfique contenu dans les particules pouvant être libéré de façon quasi immédiate, progressive et répétable sur la peau, le cheveu et les phanères au contact de l'eau

On sait qu'il existe une nécessité dans de nombreux domaines industriels, de protéger un certain nombre de molécules fragiles ou volatiles et de contrôler leur relargage vers un milieu extérieur.

Un des moyens permettant d'atteindre un tel but est leur encapsulation. Cette encapsulation a pour objectif de diminuer l'évaporation et le transfert vers l'environnement de la matière active, soit au cours du stockage, soit au cours de l'élaboration des produits ou encore au cours de leur utilisation. Elle peut également permettre de rendre le matériau plus facile d'utilisation en le diluant et en favorisant sa répartition homogène au sein du support.

La micro-encapsulation regroupe l'ensemble des technologies permettant l'enrobage ou le piégeage de principes actifs sous forme solide, liquide, ou gazeuse au sein de particules individualisées dont la taille s'échelonne entre quelques microns et quelques millimètres. Si ces microparticules sont creuses (vésiculaires) on parle de microcapsules, si elles sont pleines (matricielles) on parle de microsphères. Leur taille varie de 1$\mu$m à plus de 1000$\mu$m. Ces microparticules peuvent être biodégradables ou non et peuvent contenir entre 5 et 90% (en masse) de substance active.

Les substances actives encapsulées sont d'origines très variées: principes actifs pharmaceutiques, cosmétiques, additifs alimentaires, produits phytosanitaires, essences parfumées, micro-organismes, cellules, ou encore catalyseurs de réaction chimique...

Tout l'intérêt des microparticules d'encapsulation réside dans la présence d'une membrane polymérique, qui isole et protège le contenu du milieu extérieur. Selon les cas, la membrane sera détruite lors de l'utilisation pour libérer son contenu (exemple : encarts publicitaires "scratch and sniff" libérant le parfum lorsqu'on écrase les microcapsules), ou bien la membrane restera présente tout le long de la libération du contenu, dont elle contrôlera la vitesse de diffusion (exemple: encapsulation de médicaments pour libération ralentie).

Les matériaux enrobants sont généralement des polymères d'origine naturelle ou synthétique, hydrophobes ou hydrophiles, ou bien des lipides.

Les principaux procédés pour réaliser l'encapsulation de substances dans des microparticules sont la polymérisation interfaciale, la réticulation interfaciale, l'émulsion suivie d'une évaporation ou d'une extraction du solvant, la double émulsion évaporation/extraction de solvant, le spray-drying, le prilling, la coacervation.

Dans le brevet US 5 508 259, on a proposé des compositions parfumantes non aqueuses, comprenant des parfums encapsulés dans des capsules solubles dans l'eau. Lesdites capsules sont obtenues par des techniques d'encapsulation conventionnelles et en particulier le spray-drying d'une émulsion constituée d'un substrat solide filmogène en combinaison avec un agent émulsionnant et un mélange d'ingrédients de parfumerie. Le substrat solide filmogène est notamment choisi parmi l'acétate de polyvinyle, l'alcool polyvinylique, des dextrines, de l'amidon naturel ou modifié, les gommes végétales, les pectines, les xanthanes, les alginates, carraghénanes ou encore les dérivés de cellulose tels que par exemple la carboxyméthylcellulose, la méthylcellulose ou l'hydroxyéthylcellulose. L'émulsion est ensuite déshydratée par un procédé classique d'atomisation (spray-drying), qui consiste, tel que décrit à l'exemple 1, à la pulvériser en fines gouttelettes dans un atomiseur avec un débit de 50kg/h et une pression de 0,45 bars, au contact d'un courant d'air de 320m$^3$/h chauffé à 350°C afin d'évaporer l'eau, ce qui permet d'obtenir une poudre fine avec un diamètre de particules compris entre 20 et 80microns et contenant 20% en poids de parfum.

Cependant, on a remarqué que les particules obtenues par ce procédé étaient fortement odorantes à l'état sec à cause de la présence de parfum libre (non encapsulé), qu'elles étaient principalement constituées d'agglomérats pouvant nuire à l'homogénéité du produit et gêner la bonne application du produit et ne possédaient pas les caractéristiques de densité adaptées à l'objectif de l'invention.

Dans le brevet US 6 200 949, on a décrit également un procédé de formation d'une matière particulaire contenant un parfum hydrophile comprenant les étapes successives consistant à former une émulsion aqueuse de parfum contenant 40 à 60% en poids d'eau, 3 à 30% en poids de maltodextrine, 10 à 40% en poids d'amidon modifié hydrophobe, puis à la sécher par pulvérisation dans un atomiseur (courant d'air de 420m$^3$/h chauffé à 204°C de sorte que les particules sont formées avec une taille moyenne d'environ 3 à environ 10 microns et une teneur en parfum de 15 à 50% en poids. Cependant, les particules obtenues par ce procédé sont fortement odorantes à l'état sec à cause de la présence de parfum libre (non encapsulé), sont principalement constituées d'agglomérats, peuvent nuire à l'homogénéité du produit et ne possèdent pas les caractéristiques de densité adaptées à l'objectif de l'invention.

Il est donc très important de pouvoir disposer de particules d'encapsulation étanches qui ne libèrent leur contenu qu'à la demande (en réponse à l'humidité ambiante, notamment dans les zones climatiques humides, par exemple en réponse à la transpiration corporelle, au shampooing ou sous la douche, etc...), d'une part pour garantir la protection dans le temps de l'actif encapsulé surtout s'il est fragile et/ou volatil et d'autre part pour éviter les interactions avec les autres ingrédients de la formule. Lorsque l'agent bénéfique encapsulé est un ingrédient de parfumerie et/ou un parfum complet, il est d'autant plus important que l'encapsulation soit totale, ce qui conduit à des particules inodores en formules anhydres permettant au formulateur de les associer ou non avec n'importe quel parfum libre de son choix (identique ou différent) sans risque d'interactions ou de perturbation de la note parfumée choisie.

Dans le brevet EP1917098B1, on a proposé un procédé de préparation de particules d'encapsulation par précipitation, lequel procédé emploie :

- une émulsion pouvant être pompée comprenant (i) une phase continue contenant un solvant et un soluté formant une matrice dissout dans ledit solvant et (ii) une phase dispersée ;
- un extracteur comprenant un gaz supercritique, sous-critique ou liquéfié ;

ledit solvant étant sensiblement plus soluble dans l'extracteur que ledit soluté formant une matrice, et ledit procédé comprenant les étapes successives consistant à:

a. combiner l'émulsion pouvant être pompée avec l'extracteur dans des conditions de mélange;
b. permettre la formation de produits d'encapsulation particulaires dans lesquels la phase dispersée est imbriquée dans une matrice solide du soluté formant une matrice;
c. collecter les produits d'encapsulation et les séparer de l'extracteur.

Il est indiqué que ce procédé peut être utilisé dans les industries pharmaceutique et agroalimentaire ainsi-que dans les domaines de l'agriculture, du «coating», des adhésifs et des catalyseurs. Il peut en particulier être utilisé pour encapsuler des actifs pharmaceutiques, des arômes, des enzymes, des colorants, des pesticides et des herbicides.

Après d'importantes recherches, la demanderesse a découvert de façon surprenante et inattendue qu'il était possible d'atteindre les objectifs tels qu'énoncés précédemment en utilisant, dans une composition anhydre soft-solid comprenant au moins un corps gras solide et au moins une huile, des particules comprenant un cœur contenant au moins un agent bénéfique et une enveloppe entourant le coeur; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines de D.E. allant de 4 à 20; lesdites particules présentant simultanément une densité de poudre versée allant de 300,0g/l à 600,0g/l et une densité absolue supérieure à 1,0; lesdites particules étant sphériques et ayant un diamètre moyen en nombre allant de 1 à 30 $\mu$m et un diamètre moyen en volume allant de 5 à 150 $\mu$m.

Ces particules peuvent être en particulier obtenues par le procédé tel que décrit dans le brevet EP1917098B1 commenté précédemment.

Les particules conformes à la présente invention permettent d'encapsuler des ingrédients bénéfiques, en particulier fragiles, de façon complète (encapsulation totale) sans dégradation dans des capsules qui seraient suffisamment résistantes et étanches pour pouvoir être conservées sans altération à l'abri de l'humidité et pouvant être aisément formulées et rester stables dans des compositions anhydres soft solid. Ces mêmes particules dans ce type de composition présentent de préférence une morphologie sphérique et une densité de poudre versée très faible pour conserver la texture légère et douce; elles ont également la capacité de s'ouvrir en présence d'eau pour pouvoir libérer leur agent bénéfique de façon quasi immédiate, progressive et répétable sur la peau, le cheveu et les phanères au contact de l'eau

Cette découverte est à la base de la présente invention.

La présente invention concerne une composition anhydre comprenant:

1) au moins des particules comprenant un cœur contenant au moins un agent bénéfique et une enveloppe entourant le coeur; ladite enveloppe comprenant au moins un polysaccharide modifié hydrophobe choisi parmi les $C_5$-$C_{20}$-alcényl succinates d'amidon et au moins un glucide hydrosoluble choisi parmi les maltodextrines ayant un Dextrose Equivalent allant de 4 à 20 ; lesdites particules présentant simultanément une densité de poudre versée allant de 300,0g/l à 600,0g/l et une densité absolue supérieure à 1,0 ; lesdites particules étant sphériques et ayant un diamètre moyen en nombre allant de 1 à 30 $\mu$m et un diamètre moyen en volume allant de 5 à 150 $\mu$m et

2) au moins une phase grasse comprenant au moins un corps gras solide et au moins une huile ;

ladite composition ayant une dureté mesurée à 32°C sous une humidité de 40% allant de 15kPa à 150kPa et de préférence allant de 20 kPa à 100kPa.

De préférence, la composition comprend un milieu physiologiquement acceptable.

Selon une forme particulière de l'invention, les compositions sont cosmétiques ou dermatologiques.

Selon une forme particulière de l'invention, les compositions selon l'invention peuvent être utilisées dans d'autres applications industrielles et notamment être des produits de consommation choisis parmi des produits vétérinaires notamment des produits d'hygiène et/ou le soin des animaux; des produits d'entretien ménager comme les produits pour le soin du linge (détachants), les produits d'entretien des appareils électroménagers, des produits d'entretien des sols, carrelages, bois etc; des produits d'entretien des matières textiles, des produits d'entretien pour la maroquinerie comme les chaussures, les semelles; des produits d'entretien dans l'industrie automobile.

L'invention concerne également un procédé cosmétique de soin et/ou d'hygiène et/ou de de conditionnement et/ou de parfumage et/ou de maquillage d'une matière kératinique consistant à appliquer sur ladite matière kératinique une composition telle que définie précédemment.

L'invention concerne encore un procédé de traitement cosmétique des odeurs corporelles et éventuellement de la transpiration humaine consistant à appliquer sur une matière kératinique une composition telle que définie précédemment comprenant au moins un actif déodorant et/ou un actif anti-transpirant sous forme libre et/ou sous forme encapsulée.

L'invention concerne aussi un produit de consommation, caractérisé par le fait qu'il est constitué par une composition telle que définie précédemment.

### Définitions

Par «composition anhydre», on entend au sens de la présente invention, une composition présentant une teneur en eau inférieure à 5 % en poids, de préférence inférieure à 2% en poids et de manière encore plus préférée inférieure à 1% en poids par rapport au poids de ladite composition voire encore moins de 0,5% et notamment exempte d'eau. Dans cette définition, l'eau mentionnée inclut l'eau résiduelle apportée par les ingrédients mélangés

Au sens de la présente invention, on entend désigner par «milieu physiologiquement acceptable», un milieu convenant à l'administration d'une composition par voie topique. Un milieu physiologiquement acceptable est un milieu sans odeur désagréable, et/ou sans aspect désagréable, et qui est parfaitement compatible avec la voie d'administration topique.

Par "matière kératinique", on entend la peau, le cuir chevelu, les lèvres, et/ou les phanères tels que les ongles et les fibres kératiniques, telles que par exemple, les poils, les cils, les sourcils et les cheveux.

Par «composition cosmétique», on entend au sens de l'invention toute composition appliquée sur une matière kératinique pour produire un effet non-thérapeutique d'hygiène, de soin, de conditionnement ou de maquillage contribuant à l'amélioration du bien-être et/ou à l'embellissement et/ou la modification de l'aspect de la matière kératinique sur laquelle on applique ladite composition.

Par «composition dermatologique», on entend au sens de l'invention toute composition appliquée sur une matière kératinique pour prévenir et/ou traiter un désordre ou un dysfonctionnement de ladite matière kératinique.

Par «traitement cosmétique», on entend au sens de l'invention tout effet non-thérapeutique de parfumage, d'hygiène, de soin, de conditionnement ou de maquillage contribuant à l'amélioration du bien-être et/ou à l'embellissement et/ou la modification de l'aspect ou de l'odeur de la matière kératinique sur laquelle on applique ladite composition.

Par «produit de consommation», on entend tout produit fabriqué destiné à être utilisé ou consommé dans la forme où il est commercialisé et qui n'est pas destiné à une fabrication ou modification ultérieure. Sans que les exemples soient limitatifs, les produits de consommation selon l'invention peuvent être des produits cosmétiques incluant aussi bien des formulations cosmétiques pour le soin et/ou l'hygiène et/ou le maquillage de la peau des lèvres, des ongles, des cils, des sourcils, des cheveux ou du cuir chevelu; des produits dermatologiques; des produits de parfumerie; des produits pharmaceutiques; des produits à usage vétérinaire notamment des produits d'hygiène et/ou le soin des animaux ; des produits d'entretien ménager comme les produits pour le soin et/ou le nettoyage du linge (détachants); des produits d'entretien des appareils électroménagers; les produits d'entretien des sols, carrelages, bois etc; des produits sanitaires; les produits d'entretien des matières textiles; des produits d'entretien dans la maroquinerie comme les chaussures, les semelles; des produits d'entretien dans l'industrie automobile.

Par «agent bénéfique», on entend au sens de l'invention tout composé présent dans un produit de consommation produisant un effet bénéfique perçu par le consommateur lors de son utilisation et/ou obtenu sur le produit de consommation lui-même, ledit effet bénéfique pouvant être une amélioration sensorielle ou une modification notamment visuelle et/ou olfactive et/ou tactile, une amélioration du confort et /ou de la facilité d'application, un effet esthétique, un effet hygiénique, une sensation de propreté, un effet curatif et/ou prophylactique.

Par «particules comprenant un cœur contenant au moins un agent bénéfique», on entend une particule comprenant au moins un agent bénéfique immobilisé, capturé et/ou encapsulé dans la matrice d'un système d'encapsulation ou de piégeage; ledit agent bénéfique étant libéré vers l'extérieur au fur et à mesure de la détérioration du système d'encapsulation ou de piégeage lorsque sa dégradation se produit au contact d'un milieu avec lequel il va réagir ou sous l'effet d'un stimulus tel qu'un apport d'eau.

### Densité de poudre versée (ou densité apparente non tassée)

La détermination est effectuée à température ambiante (20-25°C) et dans les conditions normales atmosphériques (1 atmosphère) à l'aide d'une éprouvette de 100ml. L'éprouvette est pesée vide puis remplie d'un volume de 100ml de poudre versée, sans tassement. La différence de masse entre éprouvette vide et remplie de 100ml de poudre donne la densité de poudre versée.

### Densité absolue

Principe de la mesure

La mesure consiste à déterminer le poids d'un échantillon du solide en poudre par simple pesée puis à mesurer le volume occupé par les particules de poudre en mesurant le volume de liquide déplacé par l'échantillon de poudre par immersion dans ce liquide. Le liquide choisi doit être peu volatil et ne doit pas être un solvant de la poudre. On choisit généralement le cyclohexane. Les mesures sont réalisées au moins deux fois.

Matériels : Une fiole jaugée de 10 ou 25ml et une balance de précision.

- $m_1$ est le poids de la fiole vide

- $m_2$ est le poids de la fiole remplie d'eau jusqu'au trait de jauge.

- $m_3$ est le poids de la fiole remplie de cyclohexane jusqu'au trait de jauge.

- $m_4$ est le poids de la fiole remplie environ au tiers de sa capacité par la poudre à analyser.

On remplit la fiole environ au tiers de sa capacité avec la poudre à analyser.

Méthode

On complète la fiole, un peu en-dessous du trait de jauge avec du cyclohexane. Afin d'éliminer complètement l'air emprisonné dans la poudre on opère comme suit:

1) on traite la fiole dans un bac à ultra-sons pendant 5 minutes
2) on ajuste le niveau du cyclohexane jusqu'au trait de jauge
3) on traite la fiole dans un bac à ultra-sons pendant 2 minutes
4) les étapes 2 et 3 sont répétées si nécessaire jusqu'à ce que le niveau du cyclohexane n'évolue plus.

$m_5$ est le poids de la fiole ainsi remplie.
Le poids de poudre analysée est égal à $m_4$-$m_1$ (pour une bonne précision ce poids doit être supérieur à 2g). La densité de l'air étant très faible par rapport à celle du solide on admet que $m_4$ - $m_1$ est égal au poids du solide constitutif de la poudre.
Le poids de cyclohexane correspondant au volume occupé par le solide (Vs) est égal à :

$$m_6 = (m_3 - m_1) - (m_5 - m_4) = \rho_{cyclo}.Vs \text{ où } \rho_{cyclo} \text{ est la densité du cyclohexane à la température du laboratoire.}$$

La densité absolue du solide constitutif de la poudre est égale à $\rho_{cyclos} = (m_4 - m_1) /Vs = \rho_{cyclo}(m_4 - m_1) / m_6$.

Si la densité du cyclohexane à la température du laboratoire n'est pas connue, on la détermine comme suit par rapport à celle de l'eau :
Soit Vf le volume jaugé de la fiole et $\rho_{eau}$ la densité de l'eau à la température du laboratoire on a :

$$\rho_{cyclo} = (m_3 - m_1) / Vf \text{ et } \rho_{eau} = (m_2 - m_1) / Vf$$

soit

$$\rho_{cyclo} = \rho_{eau} (m_2 - m_1) / (m_3 - m_1)$$

La densité absolue du solide constitutif de la poudre est égale à :

$$\rho_s = [\rho_{eau} (m_4 - m_1) (m_2 - m_1)] / [m_6 (m_3 - m_1)].$$

## DURETE

Les compositions selon l'invention qualifiées de « soft solid » ont une dureté mesurée à 32°C sous une humidité de 40% allant de 15kPa à 150kPa et de préférence allant de 20 kPa à 100kPa.
La dureté se définit comme la force maximale Fmax de contrainte mesurée en texturométrie lors de l'enfoncement d'une sonde cylindrique dans l'échantillon de formule appréciée dans des conditions de mesure précises comme suit.
Les formules sont coulées à chaud dans des pots de 9cm de diamètre et 3 cm de fond (ie : pots « Favorit Soft » de RPC Bramlage GmbH). Le refroidissement est fait à température ambiante. La dureté des formules réalisées est mesurée après 24 heures d'attente. Les pots contenant les échantillons sont caractérisés en texturométrie à l'aide d'un texturomètre tel que celui commercialisé par la société Rhéo TA-XT2, selon le protocole suivant:
A une température de 32°C et sous humidité relative de 40%, une sonde cylindrique en inox mobile de diamètre 2mm est amenée au contact de l'échantillon à une vitesse de 1mm/s. Le système de mesure détecte l'interface avec l'échantillon avec un seuil de détection égal à 0,005 newtons. La sonde s'enfonce de 0,3mm dans l'échantillon, à une vitesse de 0,1mm/s. L'appareil de mesure enregistre l'évolution de la force mesurée en compression au cours du temps, pendant la phase de pénétration. La dureté de l'échantillon correspond à la moyenne des valeurs maximales de la force détectée pendant la pénétration, sur au moins 3 mesures.

Après une mesure, le temps de relaxation est de 1 seconde et le retrait de la sonde se fait à une vitesse de 1mm/s.

La dureté de la composition est calculée par l'équation suivante :

$$dureté = \frac{F_{max}}{Surface\ du\ cylindre}$$

## PARTICULES D'ENCAPSULATION

Les particules conformes à l'invention comprennent un cœur contenant au moins un agent bénéfique et une enveloppe entourant le cœur; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines de D.E. allant de 4 à 20; lesdites particules présentant simultanément une densité de poudre versée allant de 300,0g/l à 600,0g/l et une densité absolue supérieure à 1,0; lesdites particules étant sphériques et ayant un diamètre moyen en nombre allant de 1 à 30 $\mu$m et un diamètre moyen en volume allant de 5 à 150 $\mu$m.

Les particules conformes à la présente invention sont de préférence sphériques.

Par «sphériques», on entend que la particule présente un indice de sphéricité, c'est-à-dire le rapport entre son plus grand diamètre et son plus petit diamètre, est inférieur à 1,2. Dans ce cas, de telles particules sont généralement appelées «capsules».

Par « taille moyenne » des particules on entend les paramètres D[4,3] et D[2,3] mesurés en voie sèche par diffraction laser à l'aide d'un granulomètre Microtrac S3500, les résultats étant exprimés sous la forme de distributions granulométriques en volume et en nombre donnant accès au diamètre moyen.

Les particules sphériques conformes à la présente invention présentent, de préférence, ainsi un diamètre moyen en nombre allant de 1 à 30$\mu$m plus préférentiellement allant de 2 à 15$\mu$m et encore mieux de 5 à 10$\mu$m et un diamètre moyen en volume allant de 5 à 150$\mu$m, de préférence allant de 10 à 100$\mu$m et encore mieux de 20 à 80$\mu$m.

Les particules selon l'invention contenant l'agent bénéfique représentent, de préférence, de 0,1 à 60% en poids, de préférence 0,3 à 40% en poids et encore mieux de 0,5 à 20% en poids du poids total de la composition.

## POLYSACCHARIDE MODIFIE HYDROPHOBE

On entend par «polysaccharide modifié hydrophobe» tout polysaccharide modifié par voie chimique ou enzymatique et comportant au moins un groupe fonctionnel hydrophobe.

Les polysaccharides sont des macromolécules glucidiques formées par l'enchaînement d'un grand nombre de sucres élémentaires (oses) hydrophiles liés entre eux par des liaisons O-osidiques.

Les groupes fonctionnels hydrophobes de la présente invention sont des groupes hydrocarbonés (constitués essentiellement d'atomes de carbone et d'hydrogène) comprenant de préférence au moins 6 et encore mieux au moins 8 atomes de carbone. Le nombre maximum d'atomes de carbone du groupe hydrocarboné est, de préférence, 24, plus préférentiellement 20, et encore plus préférentiellement 18. Les groupes hydrocarbonés hydrophobes peuvent être non substitués, par exemple constitués d'une simple longue chaine alkyle ou bien substitués par des groupes non réactifs comme des groupes aromatiques tels que des groupes aryles (ie: phényle) ou aralkyles (ie: benzyle) ou encore des groupes polaires tels que par exemple des carboxyles ou des hydroxyles.

Pour greffer le ou les groupe(s) fonctionnel(s) hydrophobe(s) sur les polysaccharides, on utilise généralement des dérivés des acides carboxyliques ou leurs dérivés (esters, halogénures d'acides, anhydrides).

Le polysaccharide modifié hydrophobe représente de préférence de 20 à 90% en poids, notamment 30 à 80% en poids, mieux 40 à 70% en poids, et encore mieux 40 à 60% en poids par rapport au poids total de l'enveloppe de la particule.

Les molécules d'amidons peuvent avoir comme origine botanique les céréales ou encore les tubercules. Ainsi, les amidons sont par exemple choisis parmi les amidons de maïs, de riz, de manioc, de tapioca, d'orge, de pomme de terre, de blé, de sorgho, de pois.

On entend par «amidon modifié hydrophobe» tout amidon modifié par réaction chimique ou enzymatique et comprenant au moins un groupe fonctionnel hydrophobe.

Les amidons modifiés hydrophobes conformes à l'invention sont choisis parmi

-   les $C_5$-$C_{20}$-alkyl ou $C_5$-$C_{20}$ alcényl succinates d'amidon, plus particulièrement l'octényle succinate d'amidon sodique (E1450- CAS 66829-29-6 / 52906-93-1 / 70714-61-3), en particulier celui commercialisé par National Starch sous la dénomination CAPSUL®.

On peut également citer les références commerciales CAPSUL TA®, N-LOK®, N- LOK 1930®, HI-CAP 100®, PURITY GUM 1773®, PURITY GUM 2000 ® de National Starch, CLEARGUM CO® de la société Roquette et EMCAP®, EMTEX®, DELITEX de la société Cargill.

## GLUCIDE OU POLYOL HYDROSOLUBLE

Par «glucide hydrosoluble » on entend un glucide ou polyol qui, introduit dans l'eau sans modification de pH à 25°C, à une concentration massique égale à 3%, permet l'obtention d'une solution macroscopiquement homogène et transparente, c'est à dire ayant une valeur de transmittance minimum de la lumière, à une longueur d'onde égale à 500nm, à travers un échantillon de 1cm d'épaisseur, d'au moins 80%, de préférence d'au moins 90%.

Par «glucides» (encore appelés hydrates de carbone ou carbohydrates ou saccharides) on entend l'ensemble des sucres simples ou oses et leurs combinaisons ou osides.

Les glucides comprennent habituellement:

(1) les monosaccharides ou oses qui sont de deux types : les aldoses comprenant une fonction aldéhyde sur le premier carbone et les cétoses comprenant une fonction cétone sur le deuxième carbone. On les distingue aussi suivant le nombre d'atomes de carbone qu'ils possèdent.

(2) les oligosaccharides (ou oligosides) qui sont des oligomères d'oses ayant un enchaînement de 2 à 10 unités monosaccharides unies par des liaisons glycosidiques.

(3) Les polyholosides (ou polysaccharides ou polyosides) qui sont des polymères d'oses ayant un enchaînement de monosaccharides supérieur à 10 unités

## GLUCIDES HYDROSOLUBLES

Les glucides hydrosolubles de l'invention sont choisi parmi les maltodextrines ayant une Dextrose Équivalent allant de 4 à 20.

Les maltodextrines qui sont le résultat de l'hydrolyse d'un amidon (ie : blé, maïs) ou d'une fécule (ie : pomme de terre). Elles sont constituées de différents sucres (ie : glucose, maltose, maltotriose, oligosides et polyosides) directement issus de cette réaction, dans des proportions qui dépendent du degré de l'hydrolyse.

Ce degré est mesuré par « dextrose équivalent », ou D.E., le dextrose ou D-glucose étant le résultat d'une hydrolyse totale de l'amidon. Plus le D.E. est élevé, plus l'hydrolyse est poussée, et donc plus la proportion en sucres simples (à chaîne courte) composant la maltodextrine est élevée.

Les maltodextrines utilisées conformément à l'invention sont préférentiellement de D.E. allant de 12 à 20.

On utilisera de préférence les maltodextrines de pomme de terre ou de maïs telles que celles vendues sous les dénominations commerciales MD 20P® de AVEBE, MALDEX 120®, MALDEX 170®, MALDEX 190® de Tereos.

Parmi les glucides hydrosolubles conformes à l'invention, on choisira les maltodextrines de D.E. allant de 4 à 20 et encore mieux les maltodextrines de D.E. allant de 12 à 20.

Le ou les glucides hydrosoluble(s) conformes à l'invention représentent de 10 à 80% en poids, de préférence de 15 à 70% en poids, plus préférentiellement de 20 à 65% en poids, et encore mieux de 40 à 60% en poids par rapport au poids total de l'enveloppe de la particule.

Selon l'invention, l'enveloppe des particules est constituée

-  d'au moins un $C_5$-$C_{20}$-alcényl succinate d'amidon et
-  d'au moins une maltodextrine de D.E. allant de 4 à 20 et de préférence allant de 12 à 20.

Selon une première variante, l'enveloppe des particules selon l'invention est constituée d'au moins un $C_5$-$C_{20}$-alcényl succinate d'amidon et d'au moins une maltodextrine de D.E. allant de 12 à 20.

Selon une forme particulièrement préférée de l'invention, l'enveloppe des particules d'encapsulation est constituée

a) d'au moins un $C_5$-$C_{20}$-alcényl succinate d'amidon dans une quantité allant de 20 à 90% en poids, notamment 30 à 80% en poids, de préférence 40 à 70% et encore mieux 40 à 60% en poids, par rapport au poids total de l'enveloppe de la particule.

b) et d'au moins une maltodextrine de D.E. allant de 4 à 20 dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

**Procédé de préparation des particules à libération d'agent bénéfique**

Les particules selon l'invention peuvent notamment être préparées selon le procédé décrit dans le brevet EP1917098B1 de FeyeCon.

Selon une forme particulière de l'invention, les particules sont obtenues selon un procédé comprenant au moins les étapes suivantes :

- on prépare une solution aqueuse constituée du mélange du glucide hydrosoluble choisi parmi les maltodextrines de Dextrose Equivalent allant de 4 à 20 et du polysaccharide modifié hydrophobe choisi parmi les $C_5$-$C_{20}$-alcényl succinate d'amidon puis on ajoute l'agent bénéfique et on agite de façon à former une émulsion ; et
- on homogénéise ladite émulsion ainsi formée sous haute pression à une pression allant de 10 à 200 bars et plus préférentiellement de 20 à 200 bars ; et
- on pulvérise, de préférence en continu, ladite émulsion dans une chambre de séchage ; et
- l'eau est extraite pendant une durée, de préférence, ne dépassant pas 3 heures, et plus préférentiellement ne dépassant pas 30 minutes, avec un fluide sous pression tel que le dioxyde de carbone, de préférence à l'état supercritique, de préférence à une pression d'au moins 0,3XPc et à une température d'au moins Tc-60°C avec Pc correspondant à la pression critique du gaz et Tc la température critique du gaz, de façon à obtenir des particules, de préférence sphériques, de taille moyenne, de préférence allant de 1 à 150$\mu$m, plus préférentiellement allant de 2 à 100$\mu$m et encore mieux de 5 à 80$\mu$m.

**PHASE HUILEUSE**

La phase huileuse présente dans les compositions selon l'invention comprend au moins une huile et au moins un corps gras solide.

On appelle généralement «phase huileuse», une phase liquide organique non-miscible dans l'eau. Celle-ci comprend en général un ou plusieurs composés hydrophobes qui rendent ladite phase non-miscible dans l'eau. Ladite phase est liquide (en l'absence d'agent structurant) à température ambiante (20-25°C).

**HUILES**

Par « huile », on entend un corps gras liquide à température ambiante (25 °C) et pression atmosphérique (760mm de Hg soit 105 Pa). L'huile peut être volatile ou non volatile.

Par «huile volatile», on entend au sens de l'invention une huile susceptible de s'évaporer au contact de la peau ou de la fibre kératinique en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13Pa à 40 000Pa ($10^{-3}$ à 300mm de Hg), en particulier allant de 1,3Pa à 13 000Pa (0,01 à 100mm de Hg), et plus particulièrement allant de 1,3Pa à 1300Pa (0,01 à 10mm de Hg).

Par «huile non volatile», on entend une huile restant sur la peau ou la fibre kératinique à température ambiante et pression atmosphérique au moins plusieurs heures et ayant notamment une pression de vapeur inférieure à $10^{-3}$ mm de Hg (0,13Pa).

Les huiles conformes à l'invention sont choisies de préférence parmi les huiles volatiles ou non volatiles minérales, animales, végétales, synthétiques notamment choisies parmi les huiles hydrocarbonées, les huiles fluorées, les huiles siliconées et leurs mélanges.

Par «huile hydrocarbonée», on entend une huile comportant principalement des atomes de carbone et d'hydrogène et éventuellement une ou plusieurs fonctions choisies parmi les fonctions hydroxyle, ester, éther, acide carboxylique. Généralement, l'huile présente une viscosité de 0,5 à 100 000mPa.s, de préférence de 50 à 50 000mPa.s et de préférence encore de 100 à 300 000 mPa.s.

Par «huile siliconée», on entend une huile comportant dans sa structure des atomes de carbone et au moins un atome de silicium. Par «huile fluorée», on entend une huile comportant des atomes de carbone et des atomes de fluor partiellement hydrocarbonées et/ou siliconées.

A titre d'exemple d'huiles hydrocarbonées volatiles utilisables dans l'invention, on peut citer:

- les huiles hydrocarbonées volatiles choisies parmi les huiles hydrocarbonées ayant de 8 à 16 atomes de carbones, et notamment les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars ou de Permetyls, les esters ramifiés en C8-C16, le néopentanoate d'iso-hexyle, et leurs mélanges. D'autres huiles hydrocarbonées volatiles comme les distillats de pétrole, notamment ceux vendus sous la dénomination Shell Soit par la société SHELL, peuvent aussi être utilisées; les alcanes linéaires volatils comme ceux décrits dans la demande de brevet de la société Cognis DE10 2008 012 457.

A titre d'exemple d'huile hydrocarbonée non volatile utilisable dans l'invention, on peut citer :

- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles les huiles de germe de blé, d'olive, l'huile d'amande douce, de palme, de colza, de coton, de luzerne, de pavot, de potimarron, de courge, de cassis, d'onagre, de millet, d'orge, de quinoa, de seigle, de carthame, de bancoulier, de passiflore, de rosier muscat, de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam, le squalane ;
- les éthers de synthèse ayant de 10 à 40 atomes de carbone;
- les esters de synthèse notamment d'acides gras comme les huiles de formule $R_1COOR_2$ dans laquelle R1 représente le reste d'un acide gras supérieur linéaire ou ramifié comportant de 1 à 40 atomes de carbone et $R_2$ représente une chaîne hydrocarbonée notamment ramifiée contenant de 1 à 40 atomes de carbone avec $R_1$ + R2 $\geq$ 10 comme par exemple l'huile de Purcellin (octanoate de cétostéaryle), l'isononanoate d'isononyle, le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les octanoates, décanoates ou ricinoléates d'alcools ou de polyalcools comme le dioctanoate de propylène glycol ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras liquides à température ambiante à chaîne carbonée ramifiée et/ou insaturée ayant de 12 à 26 atomes de carbone comme l'octyl dodécanol, l'alcool isostéarylique, le 2-butyloctanol, le 2-hexyl décanol, le 2-undécyl pentadécanol, l'alcool oléique ;
- les acides gras supérieurs tels que l'acide oléique, l'acide linoléique, l'acide linolénique ;
- les carbonates comme le diéthylhexylcarbonate ;
- les acétates ;
- les citrates.

A titre d'exemple d'huiles fluorées éventuellement partiellement hydrocarbonées et/ou siliconées, on peut citer les huiles fluorosiliconées, les polyéthers fluorés, les silicones fluorées telles que décrit dans le document EP-A-847752.

A titre d'exemple d'huiles siliconées non volatiles, on peut citer les polydiméthylsiloxanes (PDMS) non volatiles, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates, et leurs mélanges.

De manière préférentielle, l'huile sera choisie parmi les hydrocarbures non-volatiles linéaires ou ramifiés, d'origine minérale ou synthétique ; les carbonates ; les polydiméthylsiloxanes linéaires non volatiles (dimethicones) et leurs mélanges.

De manière encore plus préférentielle, l'huile sera choisie parmi les les polydécènes, les polydécènes hydrogénés ; les carbonates ; les polydiméthylsiloxanes linéaires non volatiles (dimethicones) et leurs mélanges.

Le ou les huiles dans la phase huileuse des compositions de l'invention sont, de préférence, présentes dans des teneurs allant de 45 à 75% et plus particulièrement de 50 à 70%par rapport au poids total de la composition.

## CORPS GRAS SOLIDES

La composition selon l'invention comprend au moins un corps gras solide choisi de préférence parmi les cires et les corps gras pâteux, et leurs mélanges et plus particulièrement les cires.

**Corps gras pâteux**

Par "corps gras pâteux" (également appelé corps gras pâteux) au sens de la présente invention, on entend un composé gras lipophile à changement d'état solide/liquide réversible, présentant à l'état solide une organisation cristalline anisotrope, et comportant à la température de 23°C une fraction liquide et une fraction solide.

En d'autres termes, la température de fusion commençante du composé pâteux peut être inférieure à 23°C. La fraction liquide du composé pâteux mesurée à 23°C peut représenter 9 à 97% en poids du composé. Cette fraction liquide à 23°C représente de préférence entre 15 et 85%, de préférence encore entre 40 et 85% en poids.

Au sens de l'invention, la température de fusion correspond à la température du pic le plus endothermique observé en analyse thermique (DSC) telle que décrite dans la norme ISO 11357-3 ; 1999. Le point de fusion d'un pâteux ou d'une cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :
Un échantillon de 5mg de pâteux ou de cire (selon le cas) disposé dans un creuset est soumis à une première montée en température allant de -20°C à 100°C, à la vitesse de chauffe de 10°C/minute, puis est refroidi de 100°C à -20°C à une vitesse de refroidissement de 10°C/minute et enfin soumis à une deuxième montée en température allant de -20°C à 100°C à une vitesse de chauffe de 5 °C/minute. Pendant la deuxième montée en température, on mesure la variation de la différence de puissance absorbée par le creuset vide et par le creuset contenant l'échantillon de pâteux ou de cire en fonction de la température. Le point de fusion du composé est la valeur de la température correspondant au sommet du pic de la courbe représentant la variation de la différence de puissance absorbée en fonction de la température.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 50 à 100%, de préférence encore de 60 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi

- la lanoline et ses dérivés
- les éthers de polyol choisi parmi les éthers de pentaérythritol et de polyalkylène glycol, les éthers d'alcool gras et de sucre, et leurs mélanges. l'éther pentaérythritol et de polyéthylène glycol comportant 5 motifs oxyéthylénés (5 OE) (nom CTFA : PEG-5 Pentaerythrityl Ether), l'éther de pentaérythritol et de polypropylène glycol comportant 5 motifs oxypropylénés (5 OP) (nom CTFA : PPG-5 Pentaerythrityl Ether), et leurs mélanges et plus spécialement le mélange PEG-5 Pentaerythrityl Ether, PPG-5 Pentaerythrityl Ether et huile de soja, commercialisé sous la dénomination « Lanolide » par la société Vevy, mélange où - les constituants se trouvent dans un rapport en poids 46/46/8: 46 % de PEG-5 Pentaerythrityl Ether, 46 % de PPG-5 Pentaerythrityl Ether et 8 % d'huile de soja.
- les composés siliconés polymères ou non
- les composés fluorés polymères ou non
- les polymères vinyliques, notamment:
- les homopolymères et les copolymères d'oléfines
- les homopolymères et copolymères de diènes hydrogénés
- les oligomères linéaires ou ramifiés, homo ou copolymères de (méth)acrylates d'alkyles ayant de préférence un groupement alkyle en $C_8$-$C_{30}$

- les oligomères homo et copolymères d'esters vinyliques ayant des groupements alkyles en $C_8$-$C_{30}$
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters,

et leurs mélanges.

Le composé pâteux est de préférence un polymère, notamment hydrocarboné.

Parmi les polyéthers liposolubles, on préfère en particulier les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les triglycérides d'acides gras et leurs dérivés
- les esters de pentaérythritol,
- les polyesters non réticulés résultant de la polycondensation entre un acide dicarboxylique ou un polyacide carboxylique linéaire ou ramifié en $C_4$-$C_{50}$ et un diol ou un polyol en $C_2$-$C_{50}$,
- les esters aliphatiques d'ester résultant de l'estérification d'un ester d'acide hydroxycarboxylique aliphatique par un acide carboxylique aliphatique. De préférence, l'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges. L'acide carboxylique aliphatique est de préférence ramifié. L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle.

L'ester d'acide hydroxy carboxylique aliphatique est choisi parmi :

a) les esters partiels ou totaux d'acides monocarboxyliques aliphatiques mono hydroxylés linéaires, saturés ;
b) les esters partiels ou totaux d'acides monocarboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en $C_2$ à $C_{16}$ ayant réagi avec un mono ou un poly acide carboxylique aliphatique mono ou poly hydroxylé, et leurs mélanges.

- les esters de dimère diol et dimère diacide, le cas échéant, estérifiés sur leur(s) fonction(s) alcool(s) ou acide(s) libre(s) par des radicaux acides ou alcools, notamment les esters dimer dilinoleate, de tels esters peuvent être notamment choisis parmi les esters de nomenclature INCI suivante : le bis-béhényl/isostéaryl/phytostéryl dimerdilinoléyle dimerdilinoléate (Plandool G), le phytostéryl/isostéryl/cétyl/stéaryl/béhényl dimerdilinoléate (Plandool H ou Plandool S), et leurs mélanges.
- les esters de rosinate hydrogénée, tel que les dimères dilinoleyl de rosinate hydrogéné (Lusplan DD-DHR ou DD-DHR de Nippon Fine Chemical)

et leurs mélanges.

**Cire(s)**

Selon un mode de réalisation préféré, la composition selon l'invention comprend au moins une cire

La cire considérée dans le cadre de la présente invention est d'une manière générale un composé lipophile, solide à température ambiante (25°C), à changement d'état solide/liquide réversible, ayant un point de fusion supérieur ou égal à 30°C pouvant aller jusqu'à 200°C et notamment jusqu'à 120°C.

En particulier, les cires convenant à l'invention peuvent présenter un point de fusion supérieur ou égal à 45°C, et en particulier supérieur ou égal à 55°C.

Les cires susceptibles d'être utilisées dans les compositions selon l'invention sont choisies parmi les cires, solides, à température ambiante d'origine animale, végétale, minérale ou de synthèse et leurs mélanges.

On peut citer par exemple les cires suivantes hydrocarbonées comprenant un chaine alkyle grasse ayant en général de 10 à 60 atomes de carbone, de préférence de 20 à 40 atomes de carbone, ladite chaîne pouvant être saturée ou insaturée, substituée ou non, linéaire, ramifiée ou cyclique, de préférence saturée et linéaire

- les alcools gras solides à température ambiante (25°C) comme l'alcool stéarylique, l'alcool cétylique ou leurs mélanges,
- les esters d'alcools gras,
- les acides gras,
- les amides d'acides gras, -
- les esters d'acides gras incluant les triglycérides,
- les éthers d'acides gras, -
- les alcools gras éthoxylés,
- les acides gras éthoxylés et leurs sels correspondants.

Parmi les alcools gras, on peut citer l'alcool stéarylique, cétéarylique ou leurs mélanges.

Parmi les esters d'alcools gras, on peut citer tri-isostearyl citrate, ethyleneglycol-di-12-hydroxystearate, tristearyl citrate, stearyl octanoate, stearyl heptanoate, tri lauryl citrate et leurs mélanges.

Parmi les esters d'acides gras, on peut citer les cires esters, les monoglycérides, les diglycérides, les triglycérides.

Comme cire ester, on peut citer le stearyl stéarate, le stearyl behenate, le stéaryl octyldodécanol, le cétéaryl béhénate, le béhényl béhénate, l'éthyleneglycol distéarate, l'éthylèneglycol dimaplimitate. On pourra utiliser en particulier un (hydroxystéaryloxy)stéarate d'alkyle en $C_{20}$-$C_{40}$ (le groupe alkyle comprenant de 20 à 40 atomes de carbone), seul ou en mélange. Une telle cire est notamment vendue sous les dénominations « Kester Wax K 82 P® », « Hydroxypolyester K 82 P® » et « Kester Wax K 80 P® » par la société KOSTER KEUNEN.

Parmi les cires triglycérides on peut citer plus particulièrement la Tribéhénine, la $C_{18}$-$C_{36}$ Triglycéride et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société Desert Whale sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone ($C_{30}$-$_{45}$ ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A-2792190.

Comme micro cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS et les micro cires de polytétrafluoroéthylène telles que celles commercialisées sous les dénominations de Microslip 519® et 519 L® par la société MICRO POWDERS.

On utilisera plus particulièrement les cires triglycérides et plus particulièrement la Tribéhénine, la $C_{18}$-$C_{36}$ Triglycéride et leurs mélanges.

La composition selon l'invention peut comprendre une teneur en corps gras solide allant de préférence de 1% à 20% en poids et en particulier de 2% à 12% en poids par rapport au poids total de la composition,

## PARTICULES D'AEROGEL DE SILICE HYDROPHOBE

Selon une forme particulière de l'invention, les compositions selon l'invention contiennent en plus des particules d'aérogel de silice hydrophobe.

La composition selon l'invention comprend des particules d'aérogel de silice hydrophobe. Celles-ci sont de préférence en dispersion dans la phase aqueuse de la composition.

Les aérogels sont des matériaux poreux ultra légers, dont les premiers ont été réalisés par Kristler en 1932.

Ils sont généralement synthétisés par un procédé sol-gel en milieu liquide puis séchés par extraction d'un fluide supercritique. Le fluide supercritique le plus communément utilisé est le $CO_2$ supercritique.

Ce type de séchage permet d'éviter la contraction des pores et du matériau.

D'autres types de séchage permettent également d'obtenir des matériaux poreux à partir de gel, à savoir (i) le séchage par cryodessiccation, consistant à solidifier à faible température le gel puis à sublimer le solvant et (ii) le séchage par évaporation. Les matériaux ainsi obtenus sont appelés respectivement cryogels et xérogels. Le procédé sol-gel et les différents séchages sont décrits en détail dans Brinker CJ., and Scherer G.W., Sol-Gel Science: New York: Academic Press, 1990.

Par «silice hydrophobe», on entend toute silice dont la surface est traitée par des agents de silylation, par exemple par des silanes halogénés tels que des alkylchlorosilanes, des siloxanes, en particulier des dimethylsiloxanes tel que l'hexamethyldisiloxane, ou des silazanes, de manière à fonctionnaliser les groupements OH par des groupements silyles Si-Rn, par exemple des groupements triméthylsilyles.

De préférence, les particules d'aérogel hydrophobe pouvant être utilisées dans la présente invention présentent avantageusement une surface spécifique par unité de masse (SM) allant de 500 à 1500m$^2$/g, de préférence de 600 à 1200m2/g et mieux de 600 à 800m$^2$/g et/ou ont une capacité d'absorption d'huile mesurée au WET POINT allant de 5 à 18ml/g de particules, de préférence de 6 à 15ml/g et mieux de 8 à 12ml/g.

La capacité d'absorption mesurée au Wet Point, et notée Wp, correspond à la quantité d'huile qu'il faut additionner à 100g de particules pour obtenir une pâte homogène.

Elle est mesurée selon la méthode dite de Wet Point ou méthode de détermination de prise d'huile de poudre selon le principe décrit dans la norme NF T 30-022. Elle correspond à la quantité d'huile adsorbée sur la surface disponible de la poudre et/ou absorbée par la poudre par mesure du Wet Point, décrite ci-dessous :

On place une quantité m= 2g de poudre sur une plaque de verre puis on ajoute goutte à goutte l'huile (isononyl isononanoate). Après addition de 4 à 5 gouttes d'huile dans la poudre, on mélange à l'aide d'une spatule et on continue d'ajouter de l'huile jusqu'à la formation de conglomérats d'huile et de poudre. A partir de ce moment, on ajoute l'huile à raison d'une goutte à la fois et on triture ensuite le mélange avec la spatule. On cesse l'addition d'huile lorsque l'on obtient une pâte ferme et lisse. Cette pâte doit se laisser étendre sur la plaque de verre sans craquelures ni formation de grumeaux. On note alors le volume Vs (exprimé en ml) d'huile utilisé.

La prise d'huile correspond au rapport Vs / m.

Les particules d'aérogel de silice hydrophobe utilisées selon la présente invention sont de préférence des particules d'aérogel de silice silylée (nom INCI silica silylate).

La préparation de particules d'aérogels de silice hydrophobe modifiées en surface par silylation est décrite plus avant dans le document US 7,470,725.

On utilisera en particulier des particules d'aérogels de silice hydrophobe modifiée en surface par groupements triméthylsilyles.

Les particules d'aérogel hydrophobe pouvant être utilisées dans la présente invention présentent avantageusement une taille, exprimée en diamètre moyen (D[0,5]), inférieure à 1500$\mu$m et de préférence allant de 1 à 30$\mu$m, de préférence de 5 à 25$\mu$m, mieux de 5 à 20$\mu$m et encore mieux de mieux de 5 à 15$\mu$m.

La surface spécifique par unité de masse peut être déterminée par la méthode d'absorption d'azote appelée méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale des particules considérées.

Les tailles des particules d'aérogel selon l'invention peuvent être mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

Selon un mode de réalisation avantageux, les particules d'aérogel hydrophobe utilisées dans la présente invention présentent une surface spécifique par unité de masse (SM) allant de 600 à 800m$^2$/g et une taille exprimée en diamètre moyen en volume (D[0,5]) allant de 5 à 20$\mu$m, mieux de 5 à 15$\mu$m.

Selon un mode de réalisation préféré, on utilisera plus particulièrement le VM-2270, dont les particules présentent une taille moyenne allant de 5 à 15 microns et une surface spécifique par unité de masse allant de 600 à 800m$^2$/g.

Les particules d'aérogel hydrophobe utilisées dans la présente invention peuvent avantageusement présenter une densité tassée p allant de 0,04g/cm$^3$ à 0,10g/cm$^3$, de préférence de 0,05g/cm$^3$ à 0,08g/cm$^3$.

Dans le cadre de la présente invention, cette densité peut être appréciée selon le protocole suivant, dit protocole de la densité tassée :

40 g de poudre sont versés dans une éprouvette graduée puis l'éprouvette est placée sur un appareil STAV 2003 de chez STAMPF VOLUMETER. L'éprouvette est ensuite soumise à une série de 2500 tassements (cette opération est recommencée jusqu'à ce que la différence de volume entre 2 essais consécutifs soit inférieure à 2 %); puis le volume final Vf de poudre tassée est mesuré directement sur l'éprouvette.

La densité tassée est déterminée par le rapport masse(m)/Vf, en l'occurrence 40/Vf (Vf étant exprimé en cm$^3$ et m en g).

Selon un mode de réalisation, les particules d'aérogel hydrophobes utilisées dans la présente invention présentent une surface spécifique par unité de volume SV allant de 5 à 60m$^2$/cm$^3$, de préférence de 10 à 50m$^2$/cm$^3$ et mieux de 15 à 40m$^2$/cm$^3$.

La surface spécifique par unité de volume est donnée par la relation :

$$SV = SM*\rho$$

où p est la densité tassée exprimée en g/cm$^3$ et SM est la surface spécifique par unité de masse exprimée en m$^2$/g, telles que définies plus haut.

Selon un mode de réalisation préféré, les particules d'aérogel hydrophobe selon l'invention présentent une surface spécifique par unité de masse (SM) allant de 500 à 1500m$^2$/g, de préférence de 600 à 1200m$^2$/g et mieux de 600 à 800m$^2$/g, et une taille exprimée en diamètre moyen (D[0,5]) allant de 1 à 30$\mu$m et/ou une capacité d'absorption d'huile mesurée au WET POINT allant de 5 à 18ml/g de particules, de préférence de 6 à 15ml/g et mieux de 8 à 12ml/g.

A titre d'aérogels de silice hydrophobe utilisables dans l'invention, on peut citer par exemple l'aérogel commercialisé sous la dénomination VM-2260 (nom INCI Silica silylate), par la société Dow Corning, dont les particules présentent une taille moyenne d'environ 1000 microns et une surface spécifique par unité de masse allant de 600 à 800m$^2$/g.

On peut également citer les aérogels commercialisés par la société Cabot sous les références AEROGEL TLD 201, AEROGEL OGD 201 et AEROGEL TLD 203, ENOVA AEROGEL MT 1100, ENOVA AEROGEL MT 1200.

On utilisera plus particulièrement l'aérogel commercialisé sous la dénomination VM-2270 (nom INCI Silica silylate), par la société Dow Corning, dont les particules présentent une taille moyenne allant de 5 à 15 microns et une surface spécifique par unité de masse allant de 600 à 800m$^2$/g.

Les particules d'aérogel de silice hydrophobes sont mises en œuvre en une teneur allant de préférence de 0,05% à 10% en poids, plus préférentiellement de 0,1% à 5% et encore plus préférentiellement de 0,5% à 4% en poids par rapport au poids total de la composition.

## ORGANOPOLYSILOXANE ELASTOMERE

Selon une forme particulière de l'invention, les compositions selon l'invention contiennent en plus au moins un organopolysiloxane élastomère

De préférence, les organopolysiloxane élastomères selon l'invention sont obtenus par réaction d'addition (a) d'un diorgano polysiloxane contenant au moins deux atomes d'hydrogène chacun lié à un atome de silicium et (b) d'un diorganopolysiloxane ayant au moins deux groupes insaturés éthyléniques lié à l'atome de silicium en présence (c) d'un catalyseur de platine selon le procédé décrit dans la demande EP-A-295886.

Selon une forme particulière de l'invention, les organopolysiloxanes élastomères sont sous forme de poudre.

Comme exemples d'organopolysiloxanes élastomères sous forme de poudre, on peut citer ayant pour nom INCI :

- DIMETHICONE/VINYL DIMETHICONE CROSSPOLYMER tels que les produits commerciaux vendus sous les noms "DOW CORNING 9505 COSMETIC POWDER", "DOW CORNING 9506 COSMETIC POWDER " par la société DOW CORNING.

Selon une forme particulière de l'invention, les organopolysiloxanes élastomères sont mélangés à au moins une huile hydrocarbonée volatile ou non volatile, et/ou au moins une huile siliconée volatile ou non volatile, pour former un gel.

Comme mélanges d'huile/organopolysiloxane élastomère non émulsionnant sous forme de gel, on peut utiliser ceux ayant les noms INCI suivants

- DIMETHICONE AND DIMETHICONE/VINYL DIMETHICONE CROSSPOLYMER comme les produits commerciaux vendus sous les noms « KSG6 », « KSG16 » par la société SHIN ETSU ,
- CYCLOPENTASILOXANE AND DIMETHICONE/VINYL DIMETHICONE CROSSPOLYMERomme les produits commerciaux vendus sous les noms "KSG-15", "KSG 24" par la société SHIN ETSU ; « Dow Corning 9040 Silicone Elastomer Blend » par la société DOW CORNING ;
- DIMETHICONE AND DIMETHICONE CROSSPOLYMER comme les produits commerciaux vendus sous les noms « Dow Corning 9041 Silicone Elastomer Blend » par la société DOW CORNING ;
- MINERAL OIL AND VINYL DIMETHICONE/LAURYL DIMETHICONE CROSSPOLYMER comme le "KSG 41" par la société SHIN ETSU
- ISODODECANE AND VINYL DIMETHICONE/LAURYL DIMETHICONE CROSSPOLYMER comme le "KSG 42" vendu par la société SHIN ETSU
- TRIETHYLHEXANOIN AND VINYL DIMETHICONE/LAURYL DIMETHICONE CROSSPOLYMER comme le « KSG 43 »vendu par la société SHIN ETSU ;
- SQUALANE AND VINYL DIMETHICONE/LAURYL DIMETHICONE CROSSPOLYMER comme le "KSG 44" vendu par la société SHIN ETSU .

On utilisera plus particulièrement les organopolysiloxanes élastomères non émulsionnants sous sous forme de gel de nom INCI : DIMETHICONE AND DIMETHICONE CROSSPOLYMER comme le produit commercial vendu sous les noms « Dow Corning 9041 Silicone Elastomer Blend » par la société DOW CORNING.

L'organopolysiloxane élastomère est de préférence présent dans la composition à des concentrations en matière active en une teneur en matière active supérieures à 1,5% en poids et notamment allant de 2 % à 8% en poids et plus préférentiellement allant de 3% à 6% en poids par rapport au poids total de la composition.

## FORMES GALENIQUES

Les compositions selon l'invention peuvent se présenter sous forme de crèmes épaisses de baume, de pommade, de gel dont la dureté peut varier en fonction de l'application souhaitée, de la zone de matière à traiter et du conditionnement souhaité.

Les compositions de l'invention peuvent être notamment conditionnées dans un pot ; dans un dispositif muni d'une paroi ajourée notamment une grille ; dans un dispositif muni d'un applicateur à billes (« roll-on ») ; sous forme de bâtonnets (sticks).

Elles contiennent à cet égard les ingrédients généralement utilisés dans ce type de produits et bien connus de l'homme de l'art.

## AGENTS BENEFIQUES

La quantité d'agent bénéfique présent dans les particules conformes à l'invention varie de préférence de 0,1 à 80% en poids du poids de la particule, de préférence 1 à 70% en poids, mieux de 10 à 60% et encore mieux de 15 à 50% en poids du poids total de la particule.

Le temps de libération de l'agent bénéfique variera évidemment selon la nature et l'intensité du stimulus.

La durée totale pour libérer l'agent bénéfique pourra être modulée et sera fortement dépendante de la composition, , de la teneur en particules, de la nature notamment chimique de l'agent bénéfique et de sa concentration dans les particules (quantité encapsulée dans la particule) et de la nature et de l'intensité du stimulus auquel sera soumise la particule contenant l'agent bénéfique. La libération peut aussi bien être quasi instantanée ou durer plusieurs heures voire plusieurs jours.

Parmi les agents bénéfiques utilisables selon l'invention, on peut citer plus particulièrement

(i) les corps gras;
(ii) les substances parfumantes;

(iii) les principes actifs pharmaceutiques;
(iv) les actifs cosmétiques.

### a) Corps gras

Ils peuvent être choisis dans le groupe comprenant

(i) les huiles naturelles d'origine végétale, animale ou marine
(ii) les huiles minérales,
(iii) les huiles hydrogénées,
(iv) les huiles de silicone,
(v) les terpènes,
(vi) le squalène,
(vii) les acides gras saturés ou insaturés,
(viii) les esters d'acide gras,
(x) les cires,
(x) les alcools gras,
(xi) les beurres comme le beurre de karité ou le beurre de cacao,
(xii) et leurs mélanges

### b) Les substances parfumantes

Par «substance parfumante», on entend tout ingrédient susceptible de dégager une odeur agréable.

Les parfums sont des compositions contenant notamment des matières premières (dénommées généralement ingrédients de parfumerie) qui sont décrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III.

Il peut s'agir de produits de synthèse ou de produits naturels, comme des huiles essentielles, des absolus, des résinoïdes, des résines, des concrètes, et/ou des produits synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage. L'huile essentielle est le plus souvent séparée de la phase aqueuse par un procédé physique n'entraînant pas de changement significatif de la composition.

Parmi les huiles essentielles utilisables selon l'invention, on peut citer celles obtenues à partir des plantes appartenant aux familles botaniques suivantes :
Abiétaceés ou Pinacées: conifères; Amaryllidacées; Anacardiacées; Anonacées: ylang; Apiacées (par exemple les ombellifères): aneth, angélique, coriandre, criste marine, carotte, persil; Aracées; Aristolochiacées; Astéracées: achilée, armoise, camomille, hélichryse; Bétulacées; Brassicacées; Burséracées: encen ; Caryophyllacées; Canellacées; Césalpiniacées: copaïfera (copahu); Chénopodacées; Cistacées: ciste; Cypéracées; Diptérocarpacées; Ericacées: gaulthérie (wintergreen); Euphorbiacées; Fabacées; Geraniacées: géranium; Guttifères; Hamamélidacées; Hernandiacées; Hypéricacées: millepertuis; Iridacées; Juglandacées; Lamiacées: thym, origan, monarde, sarriette, basilic, marjolaines, menthes, patchouli, lavandes, sauges, cataire, romarin, hysope, mélisse; Lauracées: ravensara, laurier, bois de rose, cannelle, litséa; Liliacées: ail, lys, muguet, jacinthe, jonquille,...; Magnoliacées: magnolia; Malvacées ; Méliacées; Monimiacées; Moracées: chanvre, houblon; Myricacées; Mysristicacées: muscade; Myrtacées: eucalyptus, tea tree, niaouli, cajeput, backousia, girofle, myrte; Oléacées; Pipéracées: poivre ; Pittosporacées; Poacées: citronnelle, lemongrass, vétiver; Polygonacées; Renonculacées; Rosacées: roses; Rubiacées; Rutacées: tous les citrus; Salicacées; Santalacées: santal; Saxifragacées; Schisandracées; Styracacées: benjoin; Thymélacées: bois d'agar; Tilliacées; Valérianacées: valériane, nard; Verbénacées: lantana, verveine; Violacées; Zingibéracées: galanga, curcuma, cardamome, gingembre; Zygophyllacées.

On peut citer également les huiles essentielles extraites de fleurs (lys, lavande, rose, jasmin, Ylang-Ylang, néroli), de tiges et de feuilles (patchouli, géranium, petit-grain), de fruits (framboise, pêche ,coriandre, anis, cumin, genièvre), d'écorces de fruits (bergamote, citron, orange, pamplemousse), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes ( galbanum, élémi, benjoin, myrrhe, oliban, opopanax).

Des exemples de substances parfumantes sont notamment : le géraniol, l'acétate de géranyle, le farnésol, le bornéol, l'acétate de bornyle, le linalool, l'acétate de linalyle, le propionate de linalyle, le butyrate de linalyle, le tétrahydrolinalool, le citronellol, l'acétate de citronellyle, le formiate de citronellyle, le propionate de citronellyle, le dihydromyrcénol, l'acétate de dihydromyrcényle, le tétrahydromyrcénol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzylcarbinol, l'acétate de trichlorométhylphénylcarbinyle, l'acétate de p-tert-butylcyclohexyle, l'acétate d'isononyle, l'acétate de cis-3-hexenyle , l'acétate de vétivéryle, l'acétate d'éthyle, l'acétate de butyle, l'acétate d'hexyle, l'acétate de décyle, l'acétate d'isoamyle, l'acétate de stéaryle, l'heptanoate d'allyle, le vétivérol, l'alpha-hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, l'allyl 3-cyclohexylpropionate, l'éthyl-6-(acétyloxy)-hexanoate, le caproate d'allyle, l'éthyl-2 méthyl butyrate, le méthyl dihydrojasmonate, le salicylate d'hexyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, l'anisyl propanal, le 9-décènol-1, le cis-3-hexénol, le tétrahydro-2-isobutyl-4-méthylpyrann-4-ol, 3-Methyl-5-phényl-1-pentanol, le 3a,6,6,9a-Tétraméthyl-dodécahydronaphtho[2,1-b]furanne, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, l'hexylcinnamal, le 2,4-dimethylcyclohex-3-ène-1-carbaldéhyde, le 2,6-dimethylhept-5-ènal, l'$\alpha,\alpha$-diméthyl-p-éthylphénylpropanal, le 1,3-benzodioxole-5-carboxaldéhyde, le limonène, la damascone, la décalactone, la nonalactone, le 6,6-diméthoxy-2,5,5-trimethylhex-2-ène, la 2,4,4,7-tétraméthyloct-6-èn-3-one, la1-(5,5-diméthyl-1-cyclohexenyl)-pent-4-èn-1-one, la méthylheptènone, le 4-(cyclopropylméthyl)-phénylméthyl éther, le 2-methyl-6-methylideneoct-7-en-2-ol, l'oxyde de rose, la 1-(1,2,3,4,5,6,7,8-octahydro-2,3,8,8-tetraméethyl-2-naphthyl)éthan-1-one, la 2-acétonaphthone, la 2-isopropyl-5-méthylcyclohexanone, les ionones, les méthylionones, les isométhylionones, la solanone, les irones, le cis-3-hexénol et ses esters, les muscs-indanes, les muscs-tétralines, les muscs-isochromanes, les cétones macrocycliques, les muscs-macrolactones, les muscs aliphatiques, le brassylate d'éthylène, l'essence de rose et leurs mélanges.

D'une façon générale, les parfums sont constitués d'un mélange d'ingrédients dits de parfumerie qui peuvent être également classés en notes de tête, notes de cœur et notes de fond.

Les trois notes correspondent à la volatilité plus ou moins importante des ingrédients qui les composent: note de tête fortement volatile, note de coeur moyennement volatile et note de fond faiblement volatile.

(i) La note de tête appelée aussi « départ » est celle que l'odorat perçoit en premier dès que le parfum est en contact avec la matière kératinique ou tout substrat. Mais c'est celle qui s'atténue le plus rapidement: elle ne "tient pas". Il est difficile d'exprimer le temps de persistance de cette note, car il est très variable: de quelques minutes à une dizaine de minutes

Elle est essentiellement fraîche et légère. Tous les agrumes appartiennent notamment à cette catégorie. En parfumerie, on les range sous le terme générique d'hespéridés dont font partie orange, citron, pamplemousse, bergamote, néroli etc... On citera également les aromates tels que la lavande, le laurier, le thym ou le romarin et les anisés, mentholés, aldéhydés, etc. On citera également les notes eucalyptus.

(ii) La note de cœur, parfois appelée aussi «corps» a une persistance qui va de quelques dizaines de minutes à quelques heures, mais sa principale caractéristique est de ne se révéler qu'au bout de quelques minutes. Elle "démarre" donc juste avant l'extinction de la note de tête. Elle commence à s'exprimer alors que la note de tête s'efface progressivement. Elle est représentée essentiellement par des éléments floraux, fruités ou épicés: muguet, chèvrefeuille, violette, magnolia, cannelle, géranium, jasmin, rose, iris, framboise, pêche, etc...

(iii) La note de fond, parfois appelée aussi «fond» assure la "durabilité", la persistance ou la ténacité d'un parfum. Elle est perceptible plusieurs heures, voire plusieurs jours, ou même plusieurs semaines après application sur un vêtement ou sur une mouillette ou touche olfactive, selon la concentration du parfum. On citera par exemple les bois, racines, mousses, résines et les substances animales ou minérales telles que opoponax, muscs, ambre, santal, benjoin, lichen, clou de girofle, sauge, etc. On citera également les notes vanillées, le patchouli, les coumarines...etc.

On peut bien entendu encapsuler des ingrédients appartenant à une ou plusieurs notes. Toutefois, on préférera encapsuler les ingrédients les plus volatiles (= les moins rémanents) appartenant aux notes de tête et/ou de cœur. Parmi ces ingrédients, on citera, par exemple :

Acétate de benzyle
Acétate de géranyle
Acétate de cis-3-hexenyle
Aldéhyde C18 ou nonalactone
Acétate de décyle
Allyl amyl glycolate (citral)
Acétate d'éthyle
Acétate de butyle
Allyl 3-cyclohexylpropionate
Acétate de linalyle
Alcool Phényléthylique
Acétate d'héxyle
Berryflor ou éthyl-6-(acétyloxy)-hexanoate
Acétate d'isoamyle
Caproate d'allyle
Amarocite ou 6,6-diméthoxy-2,5,5-triméthylhex-2-ène
Citral lemarome N ou 3,7-diméthylocta-2,6-diénal
Canthoxal ou anisyl propanal
Claritone ou 2,4,4,7-tétraméthyloct-6-èn-3-one
Ethyl-2méthyl butyrate
Dihydromyrcénol
Cis-3 hexenol
Hédione ou méthyl dihydrojasmonate
L-carvone
Heptanoate d'allyle
Limonène
Néobuténone alpha ou 1-(5,5-diméthyl-1-cyclohexenyl)- pent-4-èn-1-one
Méthylheptènone
Toscanol ou 4-(cyclopropylméthyl)-phénylméthyl éther
myrcenol super ou 2-methyl-6-methylideneoct-7-en-2-ol
Decalactone
Acétate de stéaryle
Oxyde de rose
Linalool
Triplai ou 2,4-dimethylcyclohex-3-ène-1-carbaldéhyde
Mélonal ou 2,6-dimethylhept-5-ènal
1-(1,2,3,4,5,6,7,8-octahydro-2,3,8,8-tetraméethyl-2-naphthyl)éthan-1-one
Hexylcinnamal
Tétrahydro-2-isobutyl-4-méthylpyrann-4-ol
Salicylate d'hexyle
1,4-Dioxacycloheptadécane-5,17-dione

et leurs mélanges

Selon une forme particulière de l'invention, on choisira de préférence les ingrédients de parfumerie ayant une pression de vapeur saturante à 25°C supérieure ou égale à 10,0Pa

La pression de vapeur saturante (ou tension de vapeur) est la pression à laquelle la phase gazeuse d'une substance est en équilibre avec sa phase liquide ou solide à une température donnée dans un système fermé. Le calcul de la pression de vapeur saturante peut se faire à l'aide de la formule suivante :

$$\ln \frac{p_{\text{sat}}}{p_0} = \frac{M.L_v}{R} \left( \frac{1}{T_0} - \frac{1}{T} \right)$$

avec :

- $T_0$ : température d'ébullition de la substance à une pression $p_0$ donnée, en degrés Kelvin,
- $p_{sat}$ : pression de vapeur saturante, dans la même unité que $p_0$
- M : masse molaire de la substance, en kg/mol
- $L_v$ : chaleur latente de vaporisation de la substance, en Joules/kg
- R : constante des gaz parfaits, égale à 8,31447 J/K/mol
- T : température de la vapeur, en K.

De préférence les substances parfumantes ayant une pression de vapeur saturante à 25°C supérieure ou égale à 10Pa représentent une quantité allant de 50 à 100% en poids, de préférence de 60 à 100% en poids, plus préférentiellement de 70 à 100% en poids, et encore mieux de 80 à 100% en poids par rapport au poids total des substances parfumantes présentes dans les particules de l'invention.

### c) Les principes actifs pharmaceutiques

On entend par «principe actif pharmaceutique» une molécule ou un mélange de molécules qui possède un effet thérapeutique, curatif et/ou prophylactique pouvant être administré par pulvérisation.

### d) Les actifs cosmétiques

On entend par «actif cosmétique» toute molécule qui possède un effet d'hygiène, de soin, de maquillage, de coloration contribuant à l'amélioration, du bien-être et/ou à l'embellissement ou la modification de l'aspect de la matière kératinique humaine sur laquelle on applique ladite composition.

Parmi les actifs cosmétiques susceptibles d'être appliqués sur des matières kératiniques humaines telles que la peau, les lèvres, le cuir chevelu, les cheveux, les cils ou les ongles, on peut citer par exemple, seuls ou en mélanges :

- les vitamines et leurs dérivés ou précurseurs, seuls ou en mélanges,
- les agents anti-oxydants;
- les agents nettoyants tels que les tensio-actifs;
- les matières colorantes ;
- les agents conditionneurs,
- les agents pour le défrisage et/ou le lissage et/ou la mise en forme des cheveux
- les agents anti-radicalaires;
- les agents photoprotecteurs comme les filtres UV organiques ou inorganiques,
- les agents autobronzants,
- les agents anti-glycation;
- les agents apaisants,
- les agents dépilatoires,
- les agents déodorants
- les agents anti-transpirants,
- les inhibiteurs de NO-synthase;
- les agents stimulant la prolifération des fibroblastes;
- les agents stimulant la prolifération des kératinocytes;
- les agents dermorelaxants,
- les agents rafraîchissants,
- les agents tenseurs,
- les agents matifiants,
- les agents anti-luisance de la peau
- les agents anti-séborrhéiques,
- les agents anti-cheveux gras,
- les agents dépigmentants
- les agents pro-pigmentants
- les agents kératolytiques,
- les agents desquamants;
- les agents hydratants,
- les agents anti-microbiens,
- les agents amincissants,
- les agents agissant sur le métabolisme énergétique des cellules,
- les agents répulsifs contre les insectes,

- les antagonistes de substances P ou de CRGP.
- les agents anti-chute des cheveux ;
- les agents anti-rides,
- les agents anti-vieillissement,
- les agents antipelliculaires.

Parmi ces actifs cosmétiques, on préférera tout particulièrement, seuls ou en mélanges

- les agents photoprotecteurs comme les filtres UV en particulier les filtres UV organiques ;
- les agents anti-luisance de la peau,
- les agents anti-séborrhéiques,
- les agents anti-cheveux gras,
- les agents déodorants,
- les agents anti-transpirants,
- les agents rafraîchissants,
- les agents matifiants,
- les agents anti-microbiens,
- les agents antipelliculaires.

Selon une forme particulièrement préférée de l'invention, le ou les agents bénéfiques présents dans les particules seront choisis parmi les substances parfumantes.

Selon une forme encore plus particulièrement préférée de l'invention, les substances parfumantes présentes dans les particules sont choisies parmi les notes de cœur et/ou les notes de tête de façon à pouvoir aussi bien compenser leur perte tout au long de la journée qu'à procurer un effet fraicheur supplémentaire au cours de la journée en réponse à la transpiration comme à l'humidité atmosphérique ou apportée par exemple par des brumisateurs.

Selon une forme particulière de l'invention, la composition contiendra

a) des particules renfermant au moins une substance parfumante et
b) au moins une substance parfumante sous forme libre, identique ou différente de la substance parfumante présente dans les dites particules.

Lesdites substances parfumantes sous forme libre peuvent être choisies parmi celles citées précédemment.

Selon une autre forme particulière de l'invention, la composition contient exclusivement la ou les substances parfumantes dans les particules d'encapsulation. Autrement dit, la totalité des ingrédients pour parfumer présents dans la composition sont contenus dans les particules.

La composition peut comprendre, en outre, d'autres ingrédients sous forme libre (non encapsulés, emprisonnés dans les particules de l'invention) utilisés couramment dans les compositions cosmétiques. De tels ingrédients peuvent être choisis parmi les antioxydants, les conservateurs, les actifs cosmétiques tels que ceux cités précédemment, les substances parfumantes telles que celles décrites précédemment, les tensioactifs, les agents d'étalement, les agents mouillants, les agents dispersants, les anti-mousses, les neutralisants, les stabilisants, les polymères et notamment les polymères filmogènes liposolubles, et leurs mélanges.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition sous forme soft-solid.

L'homme du métier pourra choisir la composition appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Selon une autre forme particulière de l'invention, les compositions selon l'invention peuvent se présenter sous forme de produit d'hygiène, en particulier des déodorants et/ou anti-transpirants où la composition comprend au moins un actif déodorant et/ou au moins un actif anti-transpirant, sous forme libre et/ou sous forme encapsulée. Plus particulièrement, les particules comprennent au moins une substance parfumante. Encore plus particulièrement, les compositions contiendront en plus au moins une substance parfumante sous forme libre, identique ou différente de la ou des substances parfumantes présentes dans les particules.

**Actif anti-transpirant**

Par «actif anti-transpirant», on entend un composé qui, à lui seul, a pour effet de diminuer le flux sudoral, et/ou de diminuer la sensation sur la peau d'humidité liée à la sueur humaine et/ou d'absorber partiellement ou totalement la sueur humaine.

Parmi les actifs antitranspirants, on peut citer les sels d'aluminium et/ou de zirconium tels que le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement le chlorhydrate d'aluminium sous forme activée ou non commercialisé par la société REHEIS sous la dénomination MICRODRY ALUMINUM CHLOROHYDRATE® ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF®, des sel d'aluminium « activés » par exemple celui commercialisé par la société REHEIS sous la dénomination REACH 103 ou par la société WESTWOOD sous la dénomination WESTCHLOR 200.

De préférence, la composition cosmétique comprend le chlorhydrate d'aluminium en tant qu'actif anti-transpirant.

Comme autre actif anti-transpirant, on peut citer les particules de perlite expansée telles que celles obtenues par le procédé d'expansion décrit dans le brevet US 5,002,698.

Les perlites utilisables selon l'invention sont généralement des aluminosilicates d'origine volcanique et ont comme composition

70,0-75,0% en poids de silice $SiO_2$
12,0-15,0% en poids d'oxyde d'aluminium oxyde $Al_2O_3$
3,0-5,0% d'oxyde de sodium $Na_2O$
3,0-5,0% d'oxyde de potassium $K_2O$
0,5-2% d'oxyde de fer $Fe_2O_3 \rightarrow$
0,2-0,7% d'oxyde de magnesium $MgO$
0,5-1,5% d'oxyde de calcium $CaO$
0,05 - 0,15% d'oxyde de titane $TiO_2$

De préférence, les particules de perlite utilisées seront broyées ; elles sont dans ce cas dites Expanded Milled Perlite (EMP). Elles ont de préférence une taille de particule définie par un diamètre médian D50 allant de 0,5 à 50$\mu$m et de préférence de 0,5 à 40$\mu$m.

De préférence, les particules de perlite utilisées présentent une densité apparente non tassée à 25°C allant de 10 et 400kg/m3 (Norme DIN 53468) et de préférence de 10 et 300kg/m3.

De préférence, les particules de perlite expansée selon l'invention ont une capacité d'absorption d'eau mesurée au WET POINT allant de 200 à 1500% et de préférence de 250 à 800%.

Le Wet Point correspond à la quantité d'eau qu'il faut additionner à 100 g de particule pour obtenir une pâte homogène. Cette méthode dérive directement de celle de la prise d'huile appliquée aux solvants. Les mesures sont faites de la même manière par l'intermédiaire du Wet Point et du Flow Point ayant respectivement comme définition suivante :

WET POINT : masse exprimée en grammes pour 100g de produit correspondant à l'obtention d'une pâte homogène lors de l'addition d'un solvant à une poudre.

FLOW POINT : masse exprimée en grammes pour 100g de produit à partir de laquelle la quantité de solvant est supérieure à la capacité de la poudre à le retenir. Cela se traduit par l'obtention d'un mélange plus ou moins homogène s'écoulant sur la plaque de verre.

Le Wet Point et le Flow point sont mesurés selon le protocole suivant :

Protocole de mesure de l'absorption d'eau.

1) Matériel utilisé

Plaque de verre (25 x 25mm)
Spatule (manche en bois et partie métallique (15 x 2,7mm)
Pinceau à poils de soie
Balance

2) Mode Opératoire

On dépose la plaque de verre sur la balance et on pèse 1g de particules de perlite. On dépose le bécher contenant le solvant ainsi que la liquipipette de prélèvement sur la balance. On ajoute progressivement le solvant à la poudre en malaxant régulièrement l'ensemble (toutes les 3 à 4 gouttes) à l'aide de la spatule

On note la masse de solvant nécessaire à l'obtention du Wet Point. On ajoute à nouveau le solvant et on note la masse permettant d'arriver au Flow Point. On effectuera la moyenne sur 3 essais.

On utilisera en particulier les particules de perlite expansée vendues sous les noms commerciaux OPTIMAT 1430 OR ou OPTIMAT 2550 par la société WORLD MINERALS.

**Actifs déodorants**

On appelle « actif déodorant », toute substance capable de masquer, absorber, améliorer et/ou réduire l'odeur désagréable résultant de la décomposition de la sueur humaine par des bactéries

Les actifs déodorants peuvent être des agents bactériostatiques ou des agents bactéricides agissant sur les germes des odeurs axillaires, comme le 2,4,4'-trichloro-2'-hydroxydiphényléther (®Triclosan), le 2,4-dichloro-2'-hydroxydiphényléther, le 3',4',5'-trichlorosalicylanilide, la 1-(3',4'-dichlorophenyl)-3-(4'-chlorophenyl)urée (®Triclocarban) ou le 3,7,11-triméthyldodéca-2,5,10-triénol (®Farnesol) ; les sels d'ammonium quaternaires comme les sels de cetyltrimethylammonium, les sels de cétylpyridinium ; les polyols comme ceux de type glycérine, 1,3-propanediol (ZEMEA PROPANEDIOL® commercialisé par Dupont Tate and Lyle Bioproducts), le 1,2-décanediol (SYMCLARIOL® de la société Symrise) ; les dérivés de glycérine comme par exemple le Caprylic/Capric Glycerides (CAPMUL MCM® de Abitec), le Caprylate ou caprate de Glycerol (DERMOSOFT GMCY® et DERMOSOFT GMC® respectivement de STRAETMANS), le Polyglyceryl-2 Caprate (DERMOSOFT DGMC® de STRAETMANS) les dérivés de biguanide comme les sels de polyhexaméthylène biguanide ; la chlorhexidine et ses sels; le 4-Phenyl-4,4-dimethyl-2butanol (SYMDEO MPP® de Symrise) ; les cyclodextrines ; les chélatants tels que le Tetrasodium Glutamate Diacetate (CAS #51981-21-6) vendu sous le nom commercial DISSOLVINE GL-47-S® de Akzo Nobel, l'EDTA (acide Ethylendiamino Tétraacétique) et le DPTA (acide 1,3-diaminopropanetétraacétique).

Parmi les actifs déodorants conformes à l'invention, on peut aussi citer également

- les sels de zinc comme le salicylate de zinc, le phénolsulfonate de zinc, le pyrrolidone carboxylate de zinc (plus communément appelé pidolate de zinc), le sulfate de zinc, le chlorure de zinc, le lactate de zinc, le gluconate de zinc, ricinoléate de zinc, glycinate de zinc, carbonate de zinc, citrate de zinc, chlorure de zinc, le laurate de zinc, l'oléate de zinc, l'orthophosphate de zinc, le stéréate de zinc, le tartrate de zinc, l'acétate de zinc ou leurs mélanges ;
- des absorbeurs d'odeurs comme les zéolites notamment métalliques sans argent, les cyclodextrines, les silicates d'oxyde métallique telles que celles décrite dans la demande US2005/063928 ; des particules d'oxyde métallique modifiées par un métal de transition telles que décrites dans les demandes US2005084464 et US2005084474, des aluminosilicates comme ceux décrits dans la demande EP1658863, des particules de dérivés de chitosan comme celles décrites dans le brevet US6916465 ;
- le bicarbonate de sodium ;
- l'acide salicylique et ses dérivés tels que l'acide n-octanoyl-5-salicylique
- l'alun.
- le triéthyl citrate

Les actifs déodorants peuvent être présents de préférence dans les compositions selon l'invention dans des concentrations pondérales allant 0,01 à 10% en poids par rapport au poids total de la composition.

La présente invention concerne également un procédé cosmétique de traitement des odeurs corporelles et éventuellement de la transpiration humaine consistant à appliquer sur la matière kératinique une composition comprenant les particules telles que définies précédemment; ladite composition comprenant au moins un actif déodorant et/ou au moins un actif anti-transpirant, sous forme libre et/ou sous forme encapsulée.

L'invention est illustrée plus en détail dans les exemples suivants.

**Exemples de préparation de particules à libération de parfum**

**Exemple A**

On a préparé des capsules en mettant en œuvre la composition suivante :

| Composition | Amidon modifié hydrophobe | Polysaccharide hydrosoluble | Parfum* | Eau |
|---|---|---|---|---|
| **Exemple A** | Amidon Capsul® de National Starch 110g | Maltodextrine de pomme de terre MD 20 P de AVEBE 110g | 55g | 225 g |
| * Le parfum utilisé a la composition suivante : | | | | |

| Ingrédients | Quantité en g |
|---|---|
| Myristate d'isopropyle | 20,5 |
| Méthyl dihydrojasmonate | 15 |
| 2-phenyléthanol | 8 |
| 1-(1,2,3,4,5,6,7,8-octahydro-2,3,8,8-tetraméthyl-2-naphthyl)éthan-1-one | 8 |
| Hexylcinnamal | 6 |
| Tétrahydro-2-isobutyl-4-méthylpyrann-4-ol | 6 |
| Hexyl Salicylate | 6 |
| Benzyl Acétate | 5 |
| 1,4-Dioxacycloheptadécane-5,17-dione | 5 |
| 3-Methyl-5-phényl-1-pentanol | 5 |
| dihydromyrcenol | 4 |
| ORANGE TERPENES 0,05% B H T (limonène >95%) | 4 |
| 2-acétonaphthone | 2 |
| 3a,6,6,9a-Tétraméthyl-dodécahydronaphtho[2,1-b]furanne | 1 |
| $\alpha,\alpha$-diméthyl-p-éthylphénylpropanal | 1 |
| 1,3-benzodioxole-5-carboxaldéhyde | 1 |
| 2-isopropyl-5-méthylcyclohexanone | 1 |
| 1-phényléthyl acetate | 0,8 |
| 2,6-dimethylhept-5-ènal (mélonal) | 0,5 |
| 2,4-dimethylcyclohex-3-ène-1-carbaldehyde (triplal) | 0,2 |

**Procédé de préparation de l'émulsion**

On a mélangé la maltodextrine de pomme de terre MD20P et l'amidon CAPSUL® (sel de sodium de l'octényle succinate d'amidon) dans l'eau jusqu'à dissolution puis on a ajouté le parfum et émulsionné avec un disperseur Ultra turrax de type Heidolph Diax 900 (moteur de puissance 900W avec une vitesse de 8000 à 26000 tour/mn électroniquement contrôlée) à la puissance maximale pendant 4 minutes

**Procédure de séchage pour obtenir des particules sphériques**

L'émulsion obtenue a été ensuite homogénéisée à une pression de 30 bars au moyen d'une pompe à haute pression puis pulvérisée dans une chambre d'atomisation au moyen d'une buse simultanément avec un courant de $CO_2$ (30 bars, 45°C) que l'on a fait circuler en continu à un débit d'environ 500g/mn pour éliminer l'eau. La poudre séchée a été retenue sur un filtre situé à la base de la chambre d'atomisation puis collectée après dépressurisation. On obtient ainsi 270g de microcapsules sphériques sous la forme d'une fine poudre blanche de granulométrie

| Exemple A | Caractéristiques mesurées des capsules | | | |
|---|---|---|---|---|
| | Taux de parfum encapsulé (%) | Taux de parfum libre (%) | Densité de poudre versée | Densité absolue |
| | 19,8 | < 0,1 | 484 | 1,12 |

**Exemples B à H (H est hors invention)**

Selon le procédé décrit à l'exemple A, on a préparé les capsules suivantes :

| Composition | Amidon modifié hydrophobe | Polysaccharide hydrosoluble | Parfum de l'exemple A | Eau |
|---|---|---|---|---|
| Exemple B | Amidon Capsul® de National Starch<br><br>110g | Maltodextrine MD 120 de Tereos<br><br><br>110g | 55g | 225 g |
| Exemple C | Amidon Capsul® de National Starch<br><br>110g | Maltodextrine MD 170 de Tereos<br><br><br>110g | 55g | 225 g |
| Exemple D | Amidon Capsul® de National Starch<br><br>110g | Maltodextrine MD 190 de Tereos<br><br><br>110g | 55g | 225 g |
| Exemple E | Amidon Capsul® de National Starch<br><br>110g | Maltodextrine de pomme de terre MD 20 P de AVEBE<br><br>110g | 105g | 225 g |
| Exemple F | Amidon Capsul® de National Starch<br><br>154g (70%) | Maltodextrine de pomme de terre MD 20 P de AVEBE<br><br>66g | 55g | 225 g |
| Exemple G | Amidon Capsul® de | Maltodextrine de pomme de terre MD 20 P de | 55g | 225 g |

| | National Starch 66g (30%) | AVEBE 154g | | |
|---|---|---|---|---|
| **Exemple H** | Amidon Capsul® de National Starch 110g | Sirop de glucose Glucodry G290 de TEREOS 110g | 55g | 225 g |

| Exemples | Caractéristiques mesurées des capsules | | | |
|---|---|---|---|---|
| | Taux de parfum encapsulé (%) | Taux de parfum libre (%) | Densité de poudre versée (g/l) | Densité absolue |
| **Exemple B** | 19,3 | <0,1 | 568 | 1,14 |
| **Exemple C** | 19,4 | <0,1 | 490 | 1,16 |
| **Exemple D** | 19,9 | <0,1 | 537 | 1,11 |
| **Exemple E** | 38 | 0,8 | 482 | 1,08 |
| **Exemple F** | 21,0 | 0,2 | 595 | 1,11 |
| **Exemple G** | 20,7 | 0,2 | 521 | 1,15 |
| **Exemple H** | 19,2 | 0,1 | 568 | 1,12 |

### Exemple I comparatif

On a réalisé des capsules ayant la même composition que l'exemple A tel décrit ci-dessus selon le procédé de l'exemple 1 du brevet US6200949 comprenant un séchage par spray-drying (atomisation) de l'émulsion.

L'émulsion est séchée par spray-drying au moyen d'un appareil du type Bowen Lab Model Dryer utilisant de l'air avec un débit de 420m$^3$/h à une température de 204°C et une température externe de 93°C et une vitesse de la turbine de 50000tr/mn.

Aspect morphologique des particules obtenues : polymorphe et formation d'agrégats

### Exemple J comparatif

On a réalisé des capsules ayant la même composition que l'exemple A tel décrit ci-dessus selon le procédé de l'exemple 1 du du brevet US5508259 comprenant un séchage par spray-drying (atomisation) de l'émulsion.

Le mélange a été séché par spray-drying avec un appareil du type CCM Sulzer avec un débit d'émulsion de 50kg/h, de l'air à un débit de 320m$^3$/h à 350°C et 0,45 bar.

Aspect morphologique des particules obtenues : polymorphe et formation d'agrégats

| Composition | Caractéristiques mesurées des capsules | | | |
|---|---|---|---|---|
| | Taux de parfum encapsulé (%) | Taux de parfum libre (%) | Densité de poudre versée | Densité absolue |
| **Exemple I (hors invention)** | 18,3 | 2,7 | 259 g/l | 1,16 |

(suite)

| Composition | Caractéristiques mesurées des capsules | | | |
|---|---|---|---|---|
| | Taux de parfum encapsulé (%) | Taux de parfum libre (%) | Densité de poudre versée | Densité absolue |
| **Exemple J (hors invention)** | 11,2 | 1,7 | 269 g/l | 1,12 |

### Exemple 1 : Produit déodorant et anti-transpirant

On a préparé une composition soft solid anhydre anti-transpirante ayant la composition suivante :

| Phase | Ingrédients | (% en poids) |
|---|---|---|
| A | TRIBEHENIN (SYNCROWAX HRC-PA®) | 5,6 |
| | C18-36 ACID TRIGLYCERIDES (SYNCROWAX HGLC-PA®) | 1,4 |
| B | ALUMINUM ZIRCONIUM TETRACHLOROHYDREX GLYCINE (REACH AZP-908®) | 18,7 |
| | HYDROGENATED POLYDECENE (SILKFLO 366 POLYDECENE®) | 24,6 |
| | PPG-14 BUTYL ETHER | 1,9 |
| | DIMETHICONE (VISCOSITE: 10 CST) (BELSIL DM 10®) | 37,2 |
| C | DIMETHICONE (and) DIMETHICONE CROSSPOLYMER (DOW CORNING 9041 SILICONE ELASTOMER BLEND®) | 1,9 |
| D | SILICA SILYLATE (DOW CORNING VM-2270 AEROGEL FINE PARTICLES®) | 1,9 |
| E | HYDROXYDE DE CALCIUM | 0,5 |
| F | CAPSULES DE PARFUM DE L'EXEMPLE A | 6,3 |
| Total | | 100,00 |

### Mode de préparation

On a pesé les deux cires (phase A) et on les a mises à fondre dans une cuve à 95°C.

On a ensuite mélangé séparément les huiles et les sels d'aluminium (phase B) à l'aide d'une spatule à température ambiante et on a divisé en 3 cette phase B.

A 1/3 de la phase B, on a ajouté la silicone élastomère (phase C) et on a homogénéisé à la spatule à température ambiante jusqu'à obtention d'un mélange homogène.

On a ensuite pesé l'aérogel de silice (phase D) dans une large capsule et on l'a ajouté avec précaution à un deuxième tiers de la phase B en mélangeant jusqu'à obtention d'un gel lisse.

On a également pesé l'hydroxyde de calcium (phase E) et on l'a introduit dans le dernier tiers de la phase B restante sous agitation ultra turrax 1300 t/mn pendant 15 mn.

Cuve ouverte, on a ajouté aux cires fondues les 3 préparations précédentes Et on a homogénéisé le mélange résultant en utilisant pales et turbine (petite vitesse) pendant 15 mn à 90°C.

On a enfin incorporé les capsules de parfum de l'exemple A, cuve ouverte puis homogénéisé sous agitation pendant 5 mn et laissé refroidir. On a ainsi obtenu un gel soft solid blanc.

**Protocole d'évaluation**

On a déposé de façon homogène environ 0,2 g de composition de l'exemple 1 sur une mouillette (référence chez Granger Veyron : 40140BCSI de taille 4cm x 14cm). Après une minute on a vérifié qu'elle ne présentait aucune odeur de parfum. Puis on a simulé la transpiration par un ajout d'eau d'environ 0,1g (trois sprays) sur la composition déposée. On a attendu 60s et on a à nouveau senti. On a constaté une forte odeur de parfum correspondant bien au parfum encapsulé. 4 heures plus tard, on a également pulvérisé la même quantité d'eau et remarqué la libération intense de parfum. 20 heures plus tard, on a réévalué encore de la même façon et on a remarqué à nouveau la libération de parfum.

Exemples C1 et C2 :

Similairement à l'exemple 1, on a préparé des soft solids anhydres anti-transpirant ayant les compositions suivantes:

| Exemple C1 | | |
|---|---|---|
| Phase | Ingrédients | (% en poids) |
| A | TRIBEHENIN | 5,6 |
| | C18-36 ACID TRIGLYCERIDES | 1,4 |
| B | ALUMINUM ZIRCONIUM TETRACHLOROHYDREX GLYCINE | 18,7 |
| | HYDROGENATED POLYDECENE | 24,6 |
| | PPG-14 BUTYL ETHER | 1,9 |
| | DIMETHICONE | 37,2 |
| C | DIMETHICONE (and) DIMETHICONE CROSSPOLYMER | 1,9 |
| D | SILICA SILYLATE | 1,9 |
| E | HYDROXYDE DE CALCIUM | 0,5 |
| F | CAPSULES DE PARFUM DE L'EXEMPLE I | 6,3 |
| Total | | 100,00 |

| Exemple C2 | | |
|---|---|---|
| Phase | Ingrédients | (% en poids) |
| A | TRIBEHENIN | 5,6 |
| | C18-36 ACID TRIGLYCERIDES | 1,4 |
| B | ALUMINUM ZIRCONIUM TETRACHLOROHYDREX GLYCINE | 18,7 |
| | HYDROGENATED POLYDECENE | 24,6 |
| | PPG-14 BUTYL ETHER | 1,9 |
| | DIMETHICONE | 37,2 |
| C | DIMETHICONE (and) DIMETHICONE CROSSPOLYMER | 1,9 |
| D | SILICA SILYLATE | 1,9 |
| E | HYDROXYDE DE CALCIUM | 0,5 |
| F | CAPSULES DE PARFUM DEL'EXEMPLE J | 6,3 |
| Total | | 100,00 |

**Protocole d'évaluation** :

On a déposé environ 0,2 g de composition sur une mouillette (référence chez Granger Veyron : 40140BCSI de taille 4cm x 14cm) et l'étalé de façon homogène en utilisant un doigt sec. On a noté le toucher du produit. Après une minute on a évalué intensité odeur de parfum. Puis on a simulé la transpiration par un ajout d'eau d'environ 0,1g (trois sprays) sur la composition déposée. On a attendu 60s et on a à nouveau senti.

| Soft Solid | Toucher | Intensité odeur AV | Intensité odeur AP |
|---|---|---|---|
| **Exemple 1** | Peu granuleux | inodore | Très forte odeur de parfum |
| **Exemple C1** | Granuleux | Forte odeur de parfum | Très forte odeur de parfum |
| **Exemple C2** | Granuleux | Forte odeur de parfum | Forte odeur de parfum |
| AV = avant ajout d'eau ; AP = après ajout d'eau ; | | | |

On a ainsi observé à $T_0$ que le soft solid de l'exemple 1 comprenant les capsules de parfum selon l'invention ne présente aucune odeur avant l'ajout d'eau contrairement aux exemples C1 et C2 (hors invention), ce qui montre que les capsules de parfum dans les exemples C1 et C2 ne sont pas étanches avant même l'ajout d'eau. Egalement on a observé que le toucher du soft solid de l'exemple 1 était significativement moins granuleux que celui des Exemples C1 ou C2.

On a également observé que le soft solid de l'exemple 1 après stimulation à l'eau, conduisait à une odeur très intense, ce qui montre une libération importante de parfum en réponse au stimuli eau.

La composition appliquée sur les aisselles laisse un dépôt sur la peau dégageant une odeur de parfum. Le parfum se libère au cours de la journée lorsque les capsules sont au contact de la transpiration.

## Exemple 2 : Produit déodorant et anti-transpirant

Similairement on a préparé une composition soft solid anhydre anti-transpirante ayant la composition suivante :

| Phase | Ingrédients | (% en poids) |
|---|---|---|
| A | TRIBEHENIN (SYNCROWAX HRC-PA®) | 5,7 |
| | C18-36 ACID TRIGLYCERIDES (SYNCROWAX HGLC-PA®) | 1,4 |
| | | |
| B | ALUMINUM ZIRCONIUM TETRACHLOROHYDREX GLYCINE (REACH AZP-908®) | 19,0 |
| | HYDROGENATED POLYDECENE (SILKFLO 366 POLYDECENE®) | 24,9 |
| | PPG-14 BUTYL ETHER | 1,9 |
| | DIMETHICONE (VISCOSITE: 10 CST) (BELSIL DM 10®) | 37,6 |
| C | DIMETHICONE (and) DIMETHICONE CROSSPOLYMER (DOW CORNING 9041 SILICONE ELASTOMER BLEND®) | 1,9 |
| D | SILICA SILYLATE (DOW CORNING VM-2270 AEROGEL FINE PARTICLES®) | 1,9 |
| E | HYDROXYDE DE CALCIUM | 0,5 |
| | Capsules de parfum de l'exemple A | 5,0 |
| F | Parfum libre A | 0,2 |

(suite)

| Phase | Ingrédients | (% en poids) |
|---|---|---|
| Total | | 100,00 |

Les capsules de parfum de l'exemple A peuvent être remplacées par les capsules des exemples B à H décrites précédemment.

La composition obtenue présente une dureté de 21 kPa mesurée selon la protocole décrit précédemment.

La composition appliquée sur les aisselles laisse un dépôt sur la peau dégageant une odeur de parfum. Le parfum se libère au cours de la journée lorsque les capsules sont au contact de la transpiration.

On a évalué l'intensité de l'odeur du parfum sur la peau à T0, T2h, T4h et T6h après application de la composition.

On a appliqué de façon homogène 0,2 g de la composition de l'exemple 2 sur la peau. Après une minute on a évalué l'intensité de l'odeur du parfum (APPLICATION) qu'on note de 0 à 10. 2, 4 et 6 heures plus tard on a réévalué à nouveau l'intensité de l'odeur (AV) avant un ajout d'eau d'environ 0,1g (trois sprays) sur la composition appliquée sur la peau. On a attendu 30 secondes puis on a évalué l'intensité de l'odeur (AP).

| Produit | Intensité odeur T0 | Intensité odeur T2h | | | Intensité odeur T4h | | | Intensité odeur T6h | | |
|---|---|---|---|---|---|---|---|---|---|---|
| | APPLICATION | AV | AP | Δ | AV | AP | Δ | AV | AP | Δ |
| Exemple 2 | 5,0 | 2,5 | 5,5 | 3 | 3,5 | 5,5 | 2,0 | 2,5 | 5,0 | 2,5 |

AV = avant ajout d'eau ; AP = après ajout d'eau ;
Δ= amplitude de différence d'intensité olfactive (AV - AP)
Echelle d'intensité odeur parfum : 0 à 10 (0= inodore; 10= odeur très intense / saturée).

On a ainsi observé à T0 que la composition de l'exemple 2 présente une odeur moyenne qui diminue fortement, étant faible à 2h après l'application. On a également observé que la pulvérisation d'eau sur le dépôt à T2h, T4h et T6h conduit à une augmentation de l'intensité de l'odeur (notamment des notes fraiches), ce qui montre une libération importante de parfum.

### Exemple 3 : Produit déodorant et anti-transpirant

De la même façon on a préparé une composition soft solid anhydre anti-transpirante ayant la composition suivante :

| Phase | Ingrédients | (% en poids) |
|---|---|---|
| A | TRIBEHENIN (SYNCROWAX HRC-PA®) | 5,8 |
| | C18-36 ACID TRIGLYCERIDES (SYNCROWAX HGLC-PA®) | 1,5 |
| B | ALUMINUM ZIRCONIUM TETRACHLOROHYDREX GLYCINE (REACH AZP-908®) | 19,4 |
| | HYDROGENATED POLYDECENE (SILKFLO 366 POLYDECENE®) | 25,5 |
| | PPG-14 BUTYL ETHER | 1,9 |
| | DIMETHICONE (VISCOSITE: 10 CST) (BELSIL DM 10®) | 38,4 |
| C | DIMETHICONE (and) DIMETHICONE CROSSPOLYMER (DOW CORNING 9041 SILICONE ELASTOMER BLEND®) | 1,9 |
| D | SILICA SILYLATE (DOW CORNING VM-2270 AEROGEL FINE PARTICLES®) | 1,9 |

(suite)

| Phase | Ingrédients | (% en poids) |
|---|---|---|
| E | HYDROXYDE DE CALCIUM | 0,5 |
| | Capsules de parfum de l'exemple A | 2,4 |
| F | Parfum libre A | 0,8 |
| Total | | 100,00 |

Les capsules de parfum de l'exemple A peuvent être remplacées par les capsules des exemples B à H décrites précédemment.

La composition obtenue présente une dureté de 16 kPa mesurée selon le protocole décrit précédemment.

On a évalué l'intensité de l'odeur du parfum sur la peau à T0, T2h, T4h et T6h après application de la composition selon le protocole décrit dans l'exemple 2.

| Produit | Intensité odeur T0 | Intensité odeur T2h | | | Intensité odeur T4h | | | Intensité odeur T6h | | |
|---|---|---|---|---|---|---|---|---|---|---|
| | APPLICATION | AV | AP | Δ | AV | AP | Δ | AV | AP | Δ |
| Exemple 3 | 7,0 | 5,0 | 7,0 | 2 | 3,5 | 6,25 | 2,75 | 3,25 | 4,75 | 1,5 |

On a ainsi observé à T0 que la composition de l'exemple 3 présente une odeur forte qui diminue étant moyenne à 2h après l'application. On a également observé à chaque temps (T2h, T4h et T6h) que la pulvérisation d'eau sur le produit conduit à une augmentation de l'intensité de l'odeur (notamment des notes fraiches), ce qui montre une libération importante de parfum.

**Revendications**

1. Composition anhydre comprenant:

    1) au moins des particules comprenant un cœur contenant au moins un agent bénéfique et une enveloppe entourant le cœur ; ladite enveloppe comprenant au moins un polysaccharide modifié hydrophobe choisi parmi les $C_5$-$C_{20}$-alcényl succinates d'amidon et au moins un glucide hydrosoluble choisi parmi les maltodextrines ayant un Dextrose Equivalent allant de 4 à 20 ;
    lesdites particules présentant simultanément une densité de poudre versée allant de 300,0g/l à 600,0g/l et une densité absolue supérieure à 1,0 ;
    lesdites particules étant sphériques et ayant un diamètre moyen en nombre allant de 1 à 30µm et un diamètre moyen en volume allant de 5 à 150µm ;
    et
    2) au moins une phase grasse comprenant au moins un corps gras solide et au moins une huile ;

    ladite composition ayant une dureté mesurée à 32°C sous une humidité de 40% allant de 15 kPa à 150 kPa et de préférence allant de 20 kPa à 100kPa.

2. Composition selon la revendication 1, comprenant un milieu physiologiquement acceptable.

3. Composition selon la revendication 1 ou 2, où les particules sont sphériques, et en particulier ont un diamètre moyen en nombre allant de 2 à 15 µm et de préférence de 5 à 10 µm et un diamètre moyen en volume allant de 10 à 100 µm et de préférence de 20 à 80 µm.

4. Composition selon l'une quelconque des revendications 1 à 3, où le polysaccharide modifié hydrophobe est l'octényle succinate d'amidon sodique.

5. Composition selon l'une quelconque des revendications 1 à 4, où le polysaccharide modifié hydrophobe représente de 20 à 90% en poids, notamment 30 à 80% en poids, mieux 40 à 70% en poids, et encore mieux 40 à 60% en poids par rapport au poids total de l'enveloppe de la particule.

6. Composition selon l'une quelconque des revendications précédentes, où le glucide hydrosoluble est choisi parmi les maltodextrines de DE allant de 12 à 20.

7. Composition selon l'une quelconque des revendications précédentes, où le ou les glucides (s) hydrosoluble(s) -représentent de 10 à 80% en poids, de préférence de 15 à 70% en poids, plus préférentiellement de 20 à 65% en poids, et encore mieux de 40 à 60% en poids par rapport au poids total de l'enveloppe de la particule.

8. Composition selon l'une quelconque des revendications précédentes, où l'enveloppe des particules à libération d'agent bénéfique est constituée

a) d'au moins un $C_5$-$C_{20}$-alcenyl succinate d'amidon dans une quantité allant de 20 à 90% en poids, notamment 30 à 80% en poids, mieux 40 à 70% en poids, et encore mieux 40 à 60% en poids par rapport au poids total de l'enveloppe de la particule et

b) au moins une maltodextrine de D.E. allant de 4 à 20 et de préférence allant de 12 à 20, dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

9. Composition selon l'une quelconque des revendications précédentes, où les particules à libération d'agent bénéfique sont susceptibles d'être obtenues selon un procédé comprenant au moins les étapes suivantes :

- on prépare une solution aqueuse constituée du mélange du glucide hydrosoluble choisi parmi les maltodextrines ayant un Dextrose Equivalent allant de 4 à 20 et du polysaccharide modifié hydrophobe choisi parmi les $C_5$-$C_{20}$-alcényl succinates d'amidon puis on ajoute l'agent bénéfique et on agite de façon à former une émulsion ; et

- on homogénéise ladite émulsion ainsi formée sous haute pression à une pression allant de 10 à 200 bars et plus préférentiellement de 20 à 200 bars ; et

- on pulvérisée ladite émulsion dans une chambre de séchage, et

- l'eau est extraite pendant une durée de préférence ne dépassant pas 3 heures, et plus préférentiellement ne dépassant pas 30 minutes, avec un fluide sous pression tel que le dioxyde de carbone, de préférence à l'état supercritique de préférence à une pression d'au moins 0,3XPc et à une température d'au moins Tc-60°C avec Pc correspondant à la pression critique du gaz et Tc la température critique du gaz, de façon à obtenir les particules à libération d'agent bénéfique.

10. Composition selon l'une quelconque des revendications précédentes, où les agents bénéfiques sont choisis parmi :

(i) les corps gras ;
(ii) les substances parfumantes ;
(iii) les principes actifs pharmaceutiques ;
(iv) les actifs cosmétiques

11. Composition selon l'une quelconque des revendications précédentes, où les agents bénéfiques sont choisis parmi les substances parfumantes et plus particulièrement celles choisies parmi les notes de cœur et/ou de tête et encore plus particulièrement choisies parmi :

Acétate de benzyle
Acétate de géranyle
Acétate de cis-3-hexenyle
Aldéhyde C18 ou nonalactone
Acétate de décyle
Allyl amyl glycolate (citral)
Acétate d'éthyle
Acétate de butyle
Allyl 3-cyclohexylpropionate
Acétate de linalyle
Alcool Phényléthylique

Acétate d'héxyle

Berryflor ou éthyl-6-(acétyloxy)-hexanoate

Acétate d'isoamyle

Caproate d'allyle

Amarocite ou 6,6-diméthoxy-2,5,5-triméthylhex-2-ène

Citral lemarome N ou 3,7-diméthylocta-2,6-diénal

Canthoxal ou anisyl propanal

Claritone ou 2,4,4,7-tétraméthyloct-6-èn-3-one

Ethyl-2méthyl butyrate

ihydromyrcénol

Cis-3 hexenol

Hédione ou méthyl dihydrojasmonate

L-carvone

Heptanoate d'allyle

Limonène

Néobuténone alpha ou 1-(5,5-diméthyl-1-cyclohexenyl)-pent-4-èn-1-one

Méthylheptènone

Toscanol ou 4-(cyclopropylméthyl)-phénylméthyl éther

myrcenol super ou 2-methyl-6-methylideneoct-7-en-2-ol

Decalactone

Acétate de stéaryle

Oxyde de rose

Linalool

Triplai ou 2,4-dimethylcyclohex-3-ène-1-carbaldéhyde

Mélonal ou 2,6-dimethylhept-5-ènal

1-(1,2,3,4,5,6,7,8-octahydro-2,3,8,8-tetraméethyl-2-naphthyl)éthan-1-one

Hexylcinnamal

Tétrahydro-2-isobutyl-4-méthylpyrann-4-ol

Salicylate d'hexyle

1,4-Dioxacycloheptadécane-5,17-dione

et leurs mélanges

**12.** Composition selon l'une quelconque des revendications précédentes, où les particules comprennent au moins une ou plusieurs substances parfumantes ayant une pression de vapeur saturante à 25°C supérieure ou égale à 10,0 Pa et, préférentiellement, la ou lesdites susbtances parfumantes représentent de 50 à 100% en poids, plus préférentiellement, de 60 à 100% en poids, encore plus préférentiellement de 70 à 100% en poids, et encore mieux de 80 à 100% en poids du poids total des substances parfumantes présentes dans les particules

**13.** Composition selon l'une quelconque des revendications précédentes, **caractérisée par le fait que**

a) les particules comprennent au moins une substance parfumante et
b) la composition comprend en plus au moins une substance parfumante sous forme libre, identique ou différente de la substance parfumante présente dans lesdites particules.

**14.** Composition selon l'une quelconque des revendications 1 à 12, **caractérisée par le fait qu'**elle contient exclusivement une ou plusieurs substances parfumantes encapsulées dans les particules.

**15.** Composition selon l'une quelconque des revendications précédentes comprenant au moins un actif déodorant et/ou au moins un actif anti-transpirant sous forme libre et/ou sous forme encapsulée et dans laquelle, plus particulièrement, les particules comprennent au moins une substance parfumante, et encore plus particulièrement, où la composition comprend en plus au moins une substance parfumante sous forme libre, identique ou différente de ladite substance parfumante présente dans lesdites particules.

**16.** Procédé de soin et/ou d'hygiène et/ou de de conditionnement et/ou de parfumage et/ou de maquillage d'une matière kératinique humaine consistant à appliquer sur ladite matière kératinique humaine une composition selon l'une quelconque des revendications précédentes.

**17.** Procédé cosmétique de traitement des odeurs corporelles et éventuellement de la transpiration humaine consistant à appliquer sur une matière kératinique une composition selon la revendication 15.

**18.** Produit de consommation, **caractérisé par le fait qu'**il est constituée par une composition telle que définie selon l'une quelconque des revendications 1 à 15.


**Patentansprüche**

**1.** Wasserfreie Zusammensetzung, umfassend

1) mindestens Teilchen mit einem Kern, der mindestens einen Effektstoff enthält, und einer den Kern umgebenden Hülle; wobei die Hülle mindestens ein hydrophob modifiziertes Polysaccharid, das aus Stärke-$C_5$-$C_{20}$-alkenylsuccinaten ausgewählt ist, und mindestens ein wasserlösliches Kohlenhydrat, das aus Maltodextrinen mit einem Dextroseäquivalent im Bereich von 4 bis 20 ausgewählt ist, umfasst; wobei die Teilchen gleichzeitig eine Pulverschüttdichte im Bereich von 300,0 g/l bis 600,0 g/l und eine absolute Dichte von mehr als 1,0 aufweisen; wobei die Teilchen kugelförmig sind und einen zahlenmittleren Durchmesser im Bereich von 1 bis 30 $\mu$m und einen volumenmittleren Durchmesser im Bereich von 5 bis 150 $\mu$m aufweisen; und 2) mindestens eine Fettphase, die mindestens eine feste Fettsubstanz und mindestens ein Öl umfasst;

wobei die Zusammensetzung eine bei 32 °C und einer Feuchtigkeit von 40 % gemessene Härte im Bereich von 15 kPa bis 150 kPa und vorzugsweise im Bereich von 20 kPa bis 100 kPa aufweist.

**2.** Zusammensetzung nach Anspruch 1, umfassend ein physiologisch unbedenkliches Medium.

**3.** Zusammensetzung nach Anspruch 1 oder 2, wobei die Teilchen kugelförmig sind und insbesondere einen zahlenmittleren Durchmesser im Bereich von 2 bis 15 $\mu$m und vorzugsweise von 5 bis 10 $\mu$m und einen volumenmittleren Durchmesser im Bereich von 10 bis 100 $\mu$m und vorzugsweise von 20 bis 80 $\mu$m aufweisen.

**4.** Zusammensetzung nach einem der Ansprüche 1 bis 3, wobei es sich bei dem hydrophob modifizierten Polysaccharid um Natriumstärkeoctenylsuccinat handelt.

**5.** Zusammensetzung nach einem der Ansprüche 1 bis 4, wobei das hydrophob modifizierte Polysaccharid 20 bis 90 Gew.-%, insbesondere 30 bis 80 Gew.-%, besser 40 bis 70 Gew.-% und noch besser 40 bis 60 Gew.-%, bezogen auf das Gesamtgewicht der Hülle des Teilchens, ausmacht.

**6.** Zusammensetzung nach einem der vorhergehenden Ansprüche, wobei das wasserlösliche Kohlenhydrat aus Maltodextrinen mit einem Dextroseäquivalent im Bereich von 12 bis 20 ausgewählt ist.

**7.** Zusammensetzung nach einem der vorhergehenden Ansprüche, wobei das wasserlösliche Kohlenhydrat bzw. die wasserlöslichen Kohlenhydrate 10 bis 80 Gew.-%, vorzugsweise 15 bis 70 Gew.-%, weiter bevorzugt 20 bis 65 Gew.-% und noch besser 40 bis 60 Gew.-%, bezogen auf das Gesamtgewicht der Hülle des Teilchens, ausmacht bzw. ausmachen.

**8.** Zusammensetzung nach einem der vorhergehenden Ansprüche, wobei die Hülle der Teilchen mit Freisetzung von Effektstoff aus

a) mindestens einem Stärke-$C_5$-$C_{20}$-alkenylsuccinat in einer Menge im Bereich von 20 bis 90 Gew.-%, insbesondere 30 bis 80 Gew.-%, besser 40 bis 70 Gew.-% und noch besser 40 bis 60 Gew.-%, bezogen auf das Gesamtgewicht der Hülle des Teilchens, und b) mindestens einem Maltodextrin mit einem D.E. im Bereich von 4 bis 20 und vorzugsweise im Bereich von 12 bis 20 in einer Menge im Bereich von 10 bis 80 Gew.-%, vorzugsweise von 15 bis 70 Gew.-%, weiter bevorzugt von 20 bis 65 Gew.-% und noch besser von 40 bis 60 Gew.-%, bezogen auf das Gesamtgewicht der Hülle des Teilchens,

besteht.

9. Zusammensetzung nach einem der vorhergehenden Ansprüche, wobei die Teilchen mit Freisetzung von Effektstoff gemäß einem Verfahren erhältlich sind, das mindestens die folgenden Schritte umfasst:

- man stellt eine wässrige Lösung her, die aus der Mischung des wasserlöslichen Kohlenhydrats, das aus Maltodextrinen mit einem Dextroseäquivalent im Bereich von 4 bis 20 ausgewählt ist, und des hydrophob modifizierten Polysaccharids, das aus Stärke-$C_5$-$C_{20}$-alkenylsuccinaten ausgewählt ist, besteht, gibt dann den Effektstoff zu und rührt zur Bildung einer Emulsion; und

- man homogenisiert die so gebildete Emulsion unter hohem Druck bei einem Druck im Bereich von 10 bis 200 bar und weiter bevorzugt von 20 bis 200 bar; und

- man versprüht die Emulsion in einer Trocknungskammer; und

- man extrahiert das Wasser über einen Zeitraum, der vorzugsweise nicht mehr als 3 Stunden und weiter bevorzugt nicht mehr als 30 Minuten beträgt, mit einem unter Druck stehenden Fluid wie Kohlendioxid, vorzugsweise in überkritischem Zustand, vorzugsweise bei einem Druck von mindestens 0,3xPc und einer Temperatur von mindestens Tc-60 °C, wobei Pc dem kritischen Druck des Gases entspricht und Tc der kritischen Temperatur des Gases entspricht, wodurch man die Teilchen mit Freisetzung von Effektstöff erhält.

10. Zusammensetzung nach einem der vorhergehenden Ansprüche, wobei die Effektstoffe aus

(i) Fettsubstanzen;

(ii) Parfümierungssubstanzen;

(iii) pharmazeutischen Wirkstoffen;

(iv) kosmetischen Wirkstoffen

ausgewählt sind.

11. Zusammensetzung nach einem der vorhergehenden Ansprüche, wobei die Effektstoffe aus Parfümierungssubstanzen ausgewählt sind und spezieller aus jenen, die aus Herz- und/oder Kopfnoten und noch spezieller aus

Benzylacetat

Geranylacetat

cis-3-Hexenylacetat

$C_{18}$-Aldehyd oder Nonalacton

Decylacetat

Allylamylglykolat (Citral)

Ethylacetat

Butylacetat

Allyl-3-cyclohexylpropionat

Linalylacetat

Phenylethylalkohol

Hexylacetat

Berryflor oder Ethyl-6-(acetyloxy)hexanoat Isoamylacetat

Allylcaproat

Amarocite oder 6,6-Dimethoxy-2,5,5-trimethylhex-2-en

Citral lemarome N oder 3,7-Dimethylocta-2,6-dienal Canthoxal oder Anisylpropanal

Claritone oder 2,4,4,7-Tetramethyloct-6-en-3-on Ethyl-2-methylbutyrat

Dihydromyrcenol

cis-3-Hexenol

Hedion oder Methyldihydrojasmonat

L-Carvon

Allylheptanoat

Limonen

Neobutenon alpha oder 1-(5,5-Dimethyl-1-cyclohexenyl)pent-4-en-1-on

Methylheptenon

Toscanol oder 4-(Cyclopropylmethyl)phenylmethylether

Myrcenol super oder 2-Methyl-6-methylidenoct-7-en-2-ol

Decalacton

Stearylacetat

Rosenoxid

Linalool

Triplal oder 2,4-Dimethylcyclohex-3-en-1-carbaldehyd
Melonal oder 2,6-Dimethylhept-5-enal
1-(1,2,3,4,5,6,7,8-Octahydro-2,3,8,8-tetramethyl-2-naphthyl)ethan-1-on
Hexylcinnamal Tetrahydro-2-isobutyl-4-methylpyran-4-ol Hexylsalicylat
1,4-Dioxacycloheptadecan-5,17-dion und Mischungen davon
ausgewählt sind.

**12.** Zusammensetzung nach einem der vorhergehenden Ansprüche, wobei die Teilchen mindestens einen oder mehrere Parfümierungssubstanzen mit einem Sättigungsdampfdruck bei 25 °C größer oder gleich 10,0 Pa aufweisen und die Parfümierungssubstanz bzw. die Parfümierungssubstanzen vorzugsweise 50 bis 100 Gew.-%, weiter bevorzugt 60 bis 100 Gew.-%, noch weiter bevorzugt 70 bis 100 Gew.-% und noch besser 80 bis 100 Gew.-% des Gesamtgewichts der in den Teilchen vorliegenden Parfümierungssubstanzen ausmacht bzw. ausmachen.

**13.** Zusammensetzung nach einem der vorhergehenden Ansprüche, **dadurch gekennzeichnet, dass**

a) die Teilchen mindestens eine Parfümierungssubstanz umfassen und
b) die Zusammensetzung außerdem mindestens eine Parfümierungssubstanz in freier Form, die mit der in den Teilchen vorliegenden Parfümierungssubstanz identisch oder davon verschieden ist, umfasst.

**14.** Zusammensetzung nach einem der Ansprüche 1 bis 12, **dadurch gekennzeichnet, dass** sie ausschließlich eine oder mehrere in den Teilchen verkapselte Parfümierungssubstanzen enthält.

**15.** Zusammensetzung nach einem der vorhergehenden Ansprüche, umfassend mindestens einen Deodorantwirkstoff und/oder mindestens einen Antitranspirantwirkstoff in freier Form und/oder in verkapselter Form, und spezieller wobei die Teilchen mindestens eine Parfümierungssubstanz umfassen und noch Spezieller wobei die Zusammensetzung außerdem mindestens eine Parfümierungssubstanz in freier Form, die mit der in den Teilchen vorliegenden Parfümierungssubstanz identisch oder davon verschieden ist, umfasst.

**16.** Verfahren zur Pflege und/oder zur Hygiene und/oder zur Konditionierung und/oder zur Parfümierung und/oder zum Schminken eines menschlichen Keratinmaterials, das darin besteht, dass man auf das menschliche Keratinmaterial eine Zusammensetzung nach einem der vorhergehenden Ansprüche aufbringt.

**17.** Kosmetisches Verfahren zur Behandlung von Körpergerüchen und gegebenenfalls menschlicher Transpiration, das darin besteht, dass man eine Zusammensetzung nach Anspruch 15 auf ein Keratinmaterial aufbringt.

**18.** Konsumprodukt, **dadurch gekennzeichnet, dass** es aus einer Zusammensetzung nach einem der Ansprüche 1 bis 15 besteht.

**Claims**

**1.** Anhydrous composition comprising:

1) at least particles comprising a core containing at least one beneficial agent and an envelope surrounding the core; said envelope comprising at least one hydrophobically modified polysaccharide chosen from starch $(C_5\text{-}C_{20})$alkenyl succinates and at least one water-soluble carbohydrate chosen from maltodextrins with a Dextrose Equivalent ranging from 4 to 20;
said particles simultaneously having a poured powder density ranging from 300.0 g/l to 600.0 g/l and an absolute density of greater than 1.0;
said particles being spherical and having a number-mean diameter ranging from 1 to 30 $\mu$m and a volume-mean diameter ranging from 5 to 150 $\mu$m;
and
2) at least one fatty phase comprising at least one solid fatty substance and at least one oil;

said composition having a hardness measured at 32°C at a humidity of 40% ranging from 15 kPa to 150 kPa and preferably ranging from 20 kPa to 100 kPa.

**2.** Composition according to Claim 1, comprising a physiologically acceptable medium.

3. Composition according to Claim 1 or 2, in which the particles are spherical and in particular have a number-mean diameter ranging from 2 to 15 $\mu$m and preferably from 5 to 10 $\mu$m and a volume-mean diameter ranging from 10 to 100 $\mu$m and preferably from 20 to 80 $\mu$m.

4. Composition according to any one of Claims 1 to 3, in which the hydrophobically modified polysaccharide is starch sodium octenyl succinate.

5. Composition according to any one of Claims 1 to 4, in which the hydrophobically modified polysaccharide represents from 20% to 90% by weight, especially from 30% to 80% by weight, better still from 40% to 70% by weight and even better still from 40% to 60% by weight relative to the total weight of the envelope of the particle.

6. Composition according to any one of the preceding claims, in which the water-soluble carbohydrate is chosen from maltodextrins with a Dextrose Equivalent ranging from 12 to 20.

7. Composition according to any one of the preceding claims, in which the water-soluble carbohydrate(s) represent from 10% to 80% by weight, preferably from 15% to 70% by weight, more preferentially from 20% to 65% by weight and even better still from 40% to 60% by weight relative to the total weight of the envelope of the particle.

8. Composition according to any one of the preceding claims, in which the envelope of the particles with release of beneficial agent is formed from

a) at least one starch ($C_5$-$C_{20}$) alkenyl succinate in an amount ranging from 20% to 90% by weight, especially from 30% to 80% by weight, better still from 40% to 70% by weight and even better still from 40% to 60% by weight relative to the total weight of the envelope of the particle and

b) at least one maltodextrin with a D.E. ranging from 4 to 20 and preferably ranging from 12 to 20, in an amount ranging from 10% to 80% by weight, preferably from 15% to 70% by weight, more preferentially from 20% to 65% by weight and even better still from 40% to 60% by weight relative to the total weight of the envelope of the particle.

9. Composition according to any one of the preceding claims, in which the particles with release of beneficial agent are capable of being obtained according to a process comprising at least the following steps:

- an aqueous solution formed from a mixture of the water-soluble carbohydrate chosen from maltodextrins with a Dextrose Equivalent ranging from 4 to 20 and of the hydrophobically modified polysaccharide chosen from starch ($C_5$-$C_{20}$) alkenyl succinates is prepared, the beneficial agent is then added and the whole is stirred so as to form an emulsion; and

- said emulsion thus formed is homogenized at high pressure at a pressure ranging from 10 to 200 bar and more preferentially from 20 to 200 bar;

- said emulsion is sprayed in a drying chamber; and

- the water is extracted for a time preferably not exceeding 3 hours, and more preferentially not exceeding 30 minutes, with a fluid under pressure such as carbon dioxide, preferably in supercritical form, preferably at a pressure of at least $0.3 \times Pc$ and at a temperature of at least Tc-60°C with Pc corresponding to the critical pressure of the gas and Tc the critical temperature of the gas, so as to obtain particles with release of beneficial agent.

10. Composition according to any one of the preceding claims, in which the beneficial agents are chosen from:

(i) fatty substances;
(ii) fragrancing substances;
(iii) pharmaceutical active principles;
(iv) cosmetic active agents.

11. Composition according to any one of the preceding claims, in which the beneficial agents are chosen from fragrancing substances and more particularly those chosen from heart notes and/or head notes and even more particularly chosen from:

benzyl acetate
geranyl acetate

cis-3-hexenyl acetate
C18 aldehyde or nonalactone
decyl acetate
allyl amyl glycolate (citral)
ethyl acetate
butyl acetate
allyl 3-cyclohexylpropionate
linalyl acetate
phenylethyl alcohol
hexyl acetate
Berryflor or ethyl 6-(acetyloxy)hexanoate isoamyl acetate
allyl caproate
Amarocite or 6,6-dimethoxy-2,5,5-trimethylhex-2-ene
Citral Lemarome N or 3,7-dimethylocta-2,6-dienal Canthoxal or anisylpropanal
Claritone or 2,4,4,7-tetramethyloct-6-en-3-one ethyl 2-methylbutyrate
dihydromyrcenol
cis-3-hexenol
Hedione or methyl dihydrojasmonate
L-carvone
allyl heptanoate
limonene
Neobutenone Alpha or 1-(5,5-dimethyl-1-cyclohoxenyl)pent-4-en-1-one
Methylheptenone
Toscanol or 4-(cyclopropylmethyl)phenyl methyl ether
Myrcenol Super or 2-methyl-6-methylideneoct-7-en-2-ol decalactone
stearyl acetate
rose oxide
linalool
Triplal or 2,4-dimethylcyclohex-3-ene-1-carbaldehyde Melonal or 2,6-dimethylhept-5-enal
1-(1,2,3,4,5,6,7,8-octahydro-2,3,8,8-tetramethyl-2-naphthyl)ethan-1-one
hexylcinnamal
tetrahydro-2-isobutyl-4-methylpyran-4-ol hexyl salicylate
1,4-dioxacycloheptadecane-5,17-dione

and mixtures thereof.

**12.** Composition according to any one of the preceding claims, in which the particles comprise at least one or more fragrancing substances with a saturation vapour pressure at 25°C of greater than or equal to 10.0 Pa and, preferentially, said fragrancing substance(s) represent from 50% to 100% by weight, more preferentially from 60% to 100% by weight, even more preferentially from 70% to 100% by weight and even better still from 80% to 100% by weight relative to the total weight of the fragrancing substances present in the particles.

**13.** Composition according to any one of the preceding claims, **characterized in that**

a) the particles comprise at least one fragrancing substance and
b) the composition also comprises at least one fragrancing substance in free form, which may be identical to or different from the fragrancing substance present in said particles.

**14.** Composition according to any one of Claims 1 to 12, **characterized in that** it exclusively contains one or more fragrancing substances encapsulated in the particles.

**15.** Composition according to any one of the preceding claims, comprising at least one deodorant active agent and/or at least one antiperspirant active agent in free form and/or in encapsulated form and in which, more particularly, the particles comprise at least one fragrancing substance, and even more particularly in which the composition also comprises at least one fragrancing substance in free form, which may be identical to or different from said fragrancing substance present in said particles.

**16.** Process for caring for and/or for the hygiene of and/or for conditioning and/or for fragrancing and/or for making up a human keratin material, which consists in applying to said human keratin material a composition according to any one of the preceding claims.

17. Cosmetic process for treating body odour and optionally human perspiration, which consists in applying to a keratin material a composition according to Claim 19.

18. Consumer product, **characterized in that** it is formed from a composition as defined according to any one of Claims 1 to 15.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5508259 A
- US 6200949 B
- EP 1917098 B1
- DE 102008012457
- EP 847752 A
- FR 2792190 A
- US 7470725 B

- EP 295886 A
- US 5002698 A
- US 2005063928 A
- US 2005084464 A
- US 2005084474 A
- EP 1658863 A
- US 6916465 B

**Littérature non-brevet citée dans la description**

- **BRINKER CJ. ; SCHERER G.W.** Sol-Gel Science. Academic Press, 1990
- *The journal of the American Chemical Society,* Février 1938, vol. 60, 309
- **VAN DE HULST, H.C.** Light Scattering by Small Particles. Wiley, 1957

- **S. ARCTANDER.** *Perfume and Flavor Chemicals,* 1969
- **S. ARCTANDER.** *Perfume and Flavor Materials of Natural Origin,* 1960
- Flavor and Fragrance Materials. Allured Publishing Co, 1991